# EUROPEAN PATENT APPLICATION

(11) **EP 3 805 394 A1**
(43) Date of publication of application: **14.04.2021**
(21) Application number: 19806476.8
(22) Date of filing: 22.05.2019
(51) Int. Cl.: C12N 15/62, A01K 67/027, C07K 14/47, C07K 19/00, C12N 5/10, C12N 9/16, C12N 15/09, C12N 15/12, C12N 15/55

(54) **METHOD FOR PRODUCING FUSION PROTEIN, NUCLEIC ACID, CELL, AND ANIMAL**

(30) Priority: 25.05.2018 JP 2018101060
(71) Applicant: University of Tsukuba, Ibaraki 305-8577 (JP)
(72) Inventor: MIZUNO, Seiya, Tsukuba-shi, Ibaraki 305-8577 (JP); SUGIYAMA, Fumihiro, Tsukuba-shi, Ibaraki 305-8577 (JP); TAKAHASHI, Satoru, Tsukuba-shi, Ibaraki 305-8577 (JP); MIZUNO, Saori, Tsukuba-shi, Ibaraki 305-8577 (JP)
(74) Representative: Ström & Gulliksson AB
(86) International application number: PCT/JP2019/020244
(87) International publication number: WO 2019/225638

(57) **Abstract**

A fusion protein including a Cas9 protein and a modifying peptide that modifies the Cas9 protein, in which the modifying peptide includes: a peptide composed of the amino acid sequence described in SEQ ID NO: 29; or a peptide composed of an amino acid sequence in which one or several amino acids are deleted, substituted, or added in the amino acid sequence described in SEQ ID NO: 29 and having activity to localize the Cas9 protein in the nucleus by forming a fusion protein with the Cas9 protein.

SEQUENCE LISTING

<110>  UNIVERSITY OF TSUKUBA

<120>  FUSION PROTEIN, NUCLEIC ACID AND METHOD OF PRODUCING CELL

<130>  PC-27508

<150>  JP2018-101060
<151>  2018-05-25

<160>  35

<170>  PatentIn version 3.5

<210>  1
<211>  104
<212>  PRT
<213>  Mus musculus

<400>  1

Pro Ser Pro Gly Gly Leu Val Ala Pro Ala Phe Thr Arg Ser Ser Ser
1               5                   10                  15

Arg Lys Arg Ala Arg Pro Pro Ala Glu Pro Gly Ser Asp Gln Pro Ala
            20                  25                  30

Pro Leu Ala Arg Arg Arg Leu Arg Leu Pro Gly Leu Asp Ser Cys Pro
        35                  40                  45

Ser Ser Leu Pro Glu Pro Ser Ser Pro Ala Glu Pro Ser Pro Pro Ala
    50                  55                  60

Asp Pro Ser Pro Pro Ala Asp Pro Gly Ser Pro Val Cys Pro Ser Pro
65                  70                  75                  80

Val Lys Arg Thr Lys Ser Thr Thr Val Tyr Val Gly Gln Gln Pro Gly
                85                  90                  95

Lys Ile Pro Ser Glu Asp Ser Val
            100

<210>  2
<211>  107
<212>  PRT
<213>  Mus musculus

<400>  2

Met Asn Leu Ser Met Lys Gln Lys Gln Glu Gly Ala Gln Glu Asn Val
1               5                   10                  15

Lys Asn Ser Pro Val Pro Arg Arg Thr Leu Lys Met Ile Gln Pro Ser
            20                  25                  30

Ala Asp Gly Ser Leu Val Gly Arg Glu Asn Glu Leu Pro Lys Gly Leu
        35                  40                  45

Phe Lys Arg Lys Leu Trp Asp Asp Gln Leu Ala Ser Gln Thr Ser Ser
    50                  55                  60

Cys Gly Pro Glu Ala Asn Glu Asn Lys Asp Val Gly Asp Leu Thr Gln
65                  70                  75                  80

Glu Ala Phe Asp Leu Ile Ser Lys Glu Asn Pro Ser Ser Gln Tyr Trp
                85                  90                  95

Lys Glu Val Ala Glu Gln Arg Arg Lys Ala Leu
            100                 105

<210>  3
<211>  91
<212>  PRT
<213>  Homo sapiens

<400>  3

Pro Ser Pro Ala Arg Pro Ala Leu Arg Ala Pro Ala Ser Ala Thr Ser
1               5                   10                  15

Gly Ser Arg Lys Arg Ala Arg Pro Pro Ala Ala Pro Gly Arg Asp Gln
            20                  25                  30

Ala Arg Pro Pro Ala Arg Arg Arg Leu Arg Leu Ser Val Asp Glu Val
        35                  40                  45

Ser Ser Pro Ser Thr Pro Glu Ala Pro Asp Ile Pro Ala Cys Pro Ser
    50                  55                  60

Pro Gly Gln Lys Ile Lys Lys Ser Thr Pro Ala Ala Gly Gln Pro Pro
65                  70                  75                  80

His Leu Thr Ser Ala Gln Asp Gln Asp Thr Ile
                85                  90

<210>  4
<211>  110
<212>  PRT
<213>  Homo sapiens

<400>  4

Met Asn Pro Ser Met Lys Gln Lys Gln Glu Glu Ile Lys Glu Asn Ile
1               5                   10                  15

Lys Asn Ser Ser Val Pro Arg Arg Thr Leu Lys Met Ile Gln Pro Ser
            20                  25                  30

Ala Ser Gly Ser Leu Val Gly Arg Glu Asn Glu Leu Ser Ala Gly Leu
        35                  40                  45

Ser Lys Arg Lys His Arg Asn Asp His Leu Thr Ser Thr Thr Ser Ser
    50                  55                  60

Pro Gly Val Ile Val Pro Glu Ser Ser Glu Asn Lys Asn Leu Gly Gly
65                  70                  75                  80

Val Thr Gln Glu Ser Phe Asp Leu Met Ile Lys Glu Asn Pro Ser Ser
                85                  90                  95

Gln Tyr Trp Lys Glu Val Ala Glu Lys Arg Arg Lys Ala Leu
            100                 105                 110

<210>  5
<211>  8818
<212>  DNA
<213>  Artificial Sequence

<220>
<223>  pX330-mG VECTOR DERIVED FROM STREPTOCOCCUS PYOGENES AND MUS
       MUSCLUS

<400>  5
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccg ggtcttcgag aagacctgtt ttagagctag aaatagcaag ttaaaataag     300

gctagtccgt tatcaacttg aaaaagtggc accgagtcgg tgcttttttg ttttagagct     360

agaaatagca agttaaaata aggctagtcc gtttttagcg cgtgcgccaa ttctgcagac     420

aaatggctct agaggtaccc gttacataac ttacggtaaa tggcccgcct ggctgaccgc     480

ccaacgaccc ccgcccattg acgtcaatag taacgccaat agggactttc cattgacgtc     540

aatgggtgga gtatttacgg taaactgccc acttggcagt acatcaagtg tatcatatgc     600

caagtacgcc ccctattgac gtcaatgacg gtaaatggcc cgcctggcat tgtgcccagt     660

acatgacctt atgggacttt cctacttggc agtacatcta cgtattagtc atcgctatta     720

ccatggtcga ggtgagcccc acgttctgct tcactctccc catctccccc ccctccccac     780

ccccaatttt gtatttattt attttttaat tattttgtgc agcgatgggg gcgggggggg     840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccccaaa gaagaagcgg aaggtcggta tccacggagt cccagcagcc gacaagaagt 1380

acagcatcgg cctggacatc ggcaccaact ctgtgggctg ggccgtgatc accgacgagt 1440

acaaggtgcc cagcaagaaa ttcaaggtgc tgggcaacac cgaccggcac agcatcaaga 1500

agaacctgat cggagccctg ctgttcgaca gcggcgaaac agccgaggcc acccggctga 1560

agagaaccgc cagaagaaga tacaccagac ggaagaaccg gatctgctat ctgcaagaga 1620

tcttcagcaa cgagatggcc aaggtggacg acagcttctt ccacagactg gaagagtcct 1680

tcctggtgga agaggataag aagcacgagc ggcaccccat cttcggcaac atcgtggacg 1740

aggtggccta ccacgagaag taccccacca tctaccacct gagaaagaaa ctggtggaca 1800

gcaccgacaa ggccgacctg cggctgatct atctggccct ggcccacatg atcaagttcc 1860

ggggccactt cctgatcgag ggcgacctga accccgacaa cagcgacgtg gacaagctgt 1920

tcatccagct ggtgcagacc tacaaccagc tgttcgagga aaaccccatc aacgccagcg 1980

gcgtggacgc caaggccatc ctgtctgcca gactgagcaa gagcagacgg ctggaaaatc 2040

tgatcgccca gctgcccggc gagaagaaga atggcctgtt cggaaacctg attgccctga 2100

gcctgggcct gacccccaac ttcaagagca acttcgacct ggccgaggat gccaaactgc 2160

agctgagcaa ggacacctac gacgacgacc tggacaacct gctggcccag atcggcgacc 2220

agtacgccga cctgtttctg gccgccaaga acctgtccga cgccatcctg ctgagcgaca 2280

tcctgagagt gaacaccgag atcaccaagg cccccctgag cgcctctatg atcaagagat 2340

acgacgagca ccaccaggac ctgaccctgc tgaaagctct cgtgcggcag cagctgcctg 2400

agaagtacaa agagattttc ttcgaccaga gcaagaacgg ctacgccggc tacattgacg 2460

gcggagccag ccaggaagag ttctacaagt tcatcaagcc catcctggaa aagatggacg 2520

gcaccgagga actgctcgtg aagctgaaca gagaggacct gctgcggaag cagcggacct 2580

tcgacaacgg cagcatcccc caccagatcc acctgggaga gctgcacgcc attctgcggc 2640

ggcaggaaga tttttaccca ttcctgaagg acaaccggga aaagatcgag aagatcctga 2700

ccttccgcat cccctactac gtgggccctc tggccagggg aaacagcaga ttcgcctgga 2760

tgaccagaaa gagcgaggaa accatcaccc cctggaactt cgaggaagtg gtggacaagg 2820

gcgcttccgc ccagagcttc atcgagcgga tgaccaactt cgataagaac ctgcccaacg 2880

agaaggtgct gcccaagcac agcctgctgt acgagtactt caccgtgtat aacgagctga 2940

ccaaagtgaa atacgtgacc gagggaatga gaaagcccgc cttcctgagc ggcgagcaga 3000

aaaaggccat cgtggacctg ctgttcaaga ccaaccggaa agtgaccgtg aagcagctga 3060

aagaggacta cttcaagaaa atcgagtgct tcgactccgt ggaaatctcc ggcgtggaag 3120

atcggttcaa cgcctccctg ggcacatacc acgatctgct gaaaattatc aaggacaagg 3180

acttcctgga caatgaggaa aacgaggaca ttctggaaga tatcgtgctg accctgacac 3240

tgtttgagga cagagagatg atcgaggaac ggctgaaaac ctatgcccac ctgttcgacg 3300

acaaagtgat gaagcagctg aagcggcgga gatacaccgg ctggggcagg ctgagccgga 3360

agctgatcaa cggcatccgg gacaagcagt ccggcaagac aatcctggat ttcctgaagt 3420

ccgacggctt cgccaacaga aacttcatgc agctgatcca cgacgacagc ctgaccttta 3480

aagaggacat ccagaaagcc caggtgtccg gccagggcga tagcctgcac gagcacattg 3540

ccaatctggc cggcagcccc gccattaaga agggcatcct gcagacagtg aaggtggtgg 3600

acgagctcgt gaaagtgatg ggccggcaca agcccgagaa catcgtgatc gaaatggcca 3660

gagagaacca gaccacccag aagggacaga agaacagccg cgagagaatg aagcggatcg 3720

aagagggcat caaagagctg ggcagccaga tcctgaaaga acaccccgtg gaaaacaccc 3780

agctgcagaa cgagaagctg tacctgtact acctgcagaa tgggcgggat atgtacgtgg 3840

accaggaact ggacatcaac cggctgtccg actacgatgt ggaccatatc gtgcctcaga 3900

gctttctgaa ggacgactcc atcgacaaca aggtgctgac cagaagcgac aagaaccggg 3960

gcaagagcga caacgtgccc tccgaagagg tcgtgaagaa gatgaagaac tactggcggc 4020

agctgctgaa cgccaagctg attacccaga gaaagttcga caatctgacc aaggccgaga 4080

gaggcggcct gagcgaactg gataaggccg gcttcatcaa gagacagctg gtggaaaccc 4140

ggcagatcac aaagcacgtg gcacagatcc tggactcccg gatgaacact aagtacgacg 4200

agaatgacaa gctgatccgg gaagtgaaag tgatcaccct gaagtccaag ctggtgtccg 4260

atttccggaa ggatttccag ttttacaaag tgcgcgagat caacaactac caccacgccc 4320

acgacgccta cctgaacgcc gtcgtgggaa ccgccctgat caaaaagtac cctaagctgg 4380

aaagcgagtt cgtgtacggc gactacaagg tgtacgacgt gcggaagatg atcgccaaga 4440

gcgagcagga aatcggcaag gctaccgcca agtacttctt ctacagcaac atcatgaact 4500

ttttcaagac cgagattacc ctggccaacg gcgagatccg gaagcggcct ctgatcgaga 4560

caaacggcga aaccggggag atcgtgtggg ataagggccg ggattttgcc accgtgcgga 4620

aagtgctgag catgccccaa gtgaatatcg tgaaaaagac cgaggtgcag acaggcggct 4680

tcagcaaaga gtctatcctg cccaagagga acagcgataa gctgatcgcc agaaagaagg 4740

actgggaccc taagaagtac ggcggcttcg acagccccac cgtggcctat tctgtgctgg 4800

tggtggccaa agtggaaaag ggcaagtcca agaaactgaa gagtgtgaaa gagctgctgg 4860

ggatcaccat catggaaaga agcagcttcg agaagaatcc catcgacttt ctggaagcca 4920

agggctacaa agaagtgaaa aaggacctga tcatcaagct gcctaagtac tccctgttcg 4980

agctggaaaa cggccggaag agaatgctgg cctctgccgg cgaactgcag aagggaaacg 5040

aactggccct gccctccaaa tatgtgaact tcctgtacct ggccagccac tatgagaagc 5100

tgaagggctc ccccgaggat aatgagcaga aacagctgtt tgtggaacag cacaagcact 5160

acctggacga gatcatcgag cagatcagcg agttctccaa gagagtgatc ctggccgacg 5220

ctaatctgga caaagtgctg tccgcctaca acaagcaccg ggataagccc atcagagagc 5280

aggccgagaa tatcatccac ctgtttaccc tgaccaatct gggagcccct gccgccttca 5340

agtactttga caccaccatc gaccggaaga ggtacaccag caccaaagag gtgctggacg 5400

ccaccctgat ccaccagagc atcaccggcc tgtacgagac acggatcgac ctgtctcagc 5460

tgggaggcga catgaatctc agtatgaagc agaagcagga gggagcccaa gagaatgtga 5520

agaatagtcc tgtcccaagg agaacgctga agatgatcca gccttctgca gatggatctc 5580

ttgttggcag agaaaatgag ttgccaaaag gcttgttcaa aaggaagctt tgggatgacc 5640

agctagcatc tcagacttca agctgtggtc cagaagctaa tgaaaataag gatgttggag 5700

acctcaccca ggaagccttt gatcttataa gtaaagagaa cccatcttct cagtattgga 5760

aagaagtggc agagcagcgg aggaaagctc tcgaattcag ccccaagaag aagagaaagg 5820

tggaggccag ctaagaattc ctagagctcg ctgatcagcc tcgactgtgc cttctagttg 5880

ccagccatct gttgtttgcc cctcccccgt gccttccttg accctggaag gtgccactcc 5940

cactgtcctt tcctaataaa atgaggaaat tgcatcgcat tgtctgagta ggtgtcattc 6000

tattctgggg ggtggggtgg ggcaggacag caagggggag gattgggaag agaatagcag 6060

gcatgctggg gagcggccgc aggaacccct agtgatggag ttggccactc cctctctgcg 6120

cgctcgctcg ctcactgagg ccgggcgacc aaaggtcgcc cgacgcccgg gctttgcccg 6180

ggcggcctca gtgagcgagc gagcgcgcag ctgcctgcag gggcgcctga tgcggtattt 6240

tctccttacg catctgtgcg gtatttcaca ccgcatacgt caaagcaacc atagtacgcg 6300

ccctgtagcg gcgcattaag cgcggcgggt gtggtggtta cgcgcagcgt gaccgctaca 6360

cttgccagcg ccctagcgcc cgctcctttc gctttcttcc cttcctttct cgccacgttc 6420

gccggctttc cccgtcaagc tctaaatcgg gggctccctt tagggttccg atttagtgct 6480

ttacggcacc tcgaccccaa aaaacttgat ttgggtgatg gttcacgtag tgggccatcg 6540

ccctgataga cggtttttcg ccctttgacg ttggagtcca cgttctttaa tagtggactc 6600

ttgttccaaa ctggaacaac actcaaccct atctcgggct attcttttga tttataaggg 6660

attttgccga tttcggccta ttggttaaaa aatgagctga tttaacaaaa atttaacgcg 6720

aattttaaca aaatattaac gtttacaatt ttatggtgca ctctcagtac aatctgctct 6780

gatgccgcat agttaagcca gccccgacac ccgccaacac ccgctgacgc gccctgacgg 6840

gcttgtctgc tcccggcatc cgcttacaga caagctgtga ccgtctccgg gagctgcatg 6900

tgtcagaggt tttcaccgtc atcaccgaaa cgcgcgagac gaaagggcct cgtgatacgc 6960

ctatttttat aggttaatgt catgataata atggtttctt agacgtcagg tggcactttt 7020

cggggaaatg tgcgcggaac ccctatttgt ttatttttct aaatacattc aaatatgtat 7080

ccgctcatga gacaataacc ctgataaatg cttcaataat attgaaaaag gaagagtatg 7140

agtattcaac atttccgtgt cgcccttatt cccttttttg cggcattttg ccttcctgtt 7200

tttgctcacc cagaaacgct ggtgaaagta aaagatgctg aagatcagtt gggtgcacga 7260

gtgggttaca tcgaactgga tctcaacagc ggtaagatcc ttgagagttt tcgccccgaa 7320

gaacgttttc caatgatgag cacttttaaa gttctgctat gtggcgcggt attatcccgt 7380

attgacgccg ggcaagagca actcggtcgc cgcatacact attctcagaa tgacttggtt 7440

gagtactcac cagtcacaga aaagcatctt acggatggca tgacagtaag agaattatgc 7500

agtgctgcca taaccatgag tgataacact gcggccaact tacttctgac aacgatcgga 7560

ggaccgaagg agctaaccgc ttttttgcac aacatggggg atcatgtaac tcgccttgat 7620

cgttgggaac cggagctgaa tgaagccata ccaaacgacg agcgtgacac cacgatgcct 7680

gtagcaatgg caacaacgtt gcgcaaacta ttaactggcg aactacttac tctagcttcc 7740

cggcaacaat taatagactg gatggaggcg gataaagttg caggaccact tctgcgctcg 7800

gcccttccgg ctggctggtt tattgctgat aaatctggag ccggtgagcg tggaagccgc 7860

ggtatcattg cagcactggg gccagatggt aagccctccc gtatcgtagt tatctacacg 7920

acggggagtc aggcaactat ggatgaacga aatagacaga tcgctgagat aggtgcctca 7980

ctgattaagc attggtaact gtcagaccaa gtttactcat atatacttta gattgattta 8040

aaacttcatt tttaatttaa aaggatctag gtgaagatcc tttttgataa tctcatgacc 8100

aaaatccctt aacgtgagtt ttcgttccac tgagcgtcag accccgtaga aaagatcaaa 8160

ggatcttctt gagatccttt ttttctgcgc gtaatctgct gcttgcaaac aaaaaaacca 8220

ccgctaccag cggtggtttg tttgccggat caagagctac caactctttt tccgaaggta 8280

actggcttca gcagagcgca gataccaaat actgtccttc tagtgtagcc gtagttaggc 8340

caccacttca agaactctgt agcaccgcct acatacctcg ctctgctaat cctgttacca 8400

gtggctgctg ccagtggcga taagtcgtgt cttaccgggt tggactcaag acgatagtta 8460

ccggataagg cgcagcggtc gggctgaacg gggggttcgt gcacacagcc cagcttggag 8520

cgaacgacct acaccgaact gagataccta cagcgtgagc tatgagaaag cgccacgctt 8580

cccgaaggga gaaaggcgga caggtatccg gtaagcggca gggtcggaac aggagagcgc 8640

acgagggagc ttccaggggg aaacgcctgg tatctttata gtcctgtcgg gtttcgccac 8700

ctctgacttg agcgtcgatt tttgtgatgc tcgtcagggg ggcggagcct atggaaaaac 8760

gccagcaacg cggccttttt acggttcctg gccttttgct ggccttttgc tcacatgt 8818

<210> 6
<211> 8816
<212> DNA
<213> Artificial Sequence

<220>
<223> pX330-mC VECTOR DERIVED FROM STREPTOCOCCUS PYOGENES AND MUS
      MUSCLUS

<400> 6
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccccaaa gaagaagcgg aaggtcggta tccacggagt cccagcagcc gacaagaagt 1380

acagcatcgg cctggacatc ggcaccaact ctgtgggctg ggccgtgatc accgacgagt 1440

acaaggtgcc cagcaagaaa ttcaaggtgc tgggcaacac cgaccggcac agcatcaaga 1500

agaacctgat cggagccctg ctgttcgaca gcggcgaaac agccgaggcc acccggctga 1560

agagaaccgc cagaagaaga tacaccagac ggaagaaccg gatctgctat ctgcaagaga 1620

tcttcagcaa cgagatggcc aaggtggacg acagcttctt ccacagactg gaagagtcct 1680

tcctggtgga agaggataag aagcacgagc ggcaccccat cttcggcaac atcgtggacg 1740

aggtggccta ccacgagaag taccccacca tctaccacct gagaaagaaa ctggtggaca 1800

gcaccgacaa ggccgacctg cggctgatct atctggccct ggcccacatg atcaagttcc 1860

ggggccactt cctgatcgag ggcgacctga accccgacaa cagcgacgtg gacaagctgt 1920

tcatccagct ggtgcagacc tacaaccagc tgttcgagga aaaccccatc aacgccagcg 1980

gcgtggacgc caaggccatc ctgtctgcca gactgagcaa gagcagacgg ctggaaaatc 2040

tgatcgccca gctgcccggc gagaagaaga atggcctgtt cggaaacctg attgccctga 2100

gcctgggcct gacccccaac ttcaagagca acttcgacct ggccgaggat gccaaactgc 2160

agctgagcaa ggacacctac gacgacgacc tggacaacct gctggcccag atcggcgacc 2220

agtacgccga cctgtttctg gccgccaaga acctgtccga cgccatcctg ctgagcgaca 2280

tcctgagagt gaacaccgag atcaccaagg cccccctgag cgcctctatg atcaagagat 2340

acgacgagca ccaccaggac ctgaccctgc tgaaagctct cgtgcggcag cagctgcctg 2400

agaagtacaa agagattttc ttcgaccaga gcaagaacgg ctacgccggc tacattgacg 2460

gcggagccag ccaggaagag ttctacaagt tcatcaagcc catcctggaa aagatggacg 2520

gcaccgagga actgctcgtg aagctgaaca gagaggacct gctgcggaag cagcggacct 2580

tcgacaacgg cagcatcccc caccagatcc acctgggaga gctgcacgcc attctgcggc 2640

ggcaggaaga tttttaccca ttcctgaagg acaaccggga aaagatcgag aagatcctga 2700

ccttccgcat cccctactac gtgggccctc tggccagggg aaacagcaga ttcgcctgga 2760

tgaccagaaa gagcgaggaa accatcaccc cctggaactt cgaggaagtg gtggacaagg 2820

gcgcttccgc ccagagcttc atcgagcgga tgaccaactt cgataagaac ctgcccaacg 2880

agaaggtgct gcccaagcac agcctgctgt acgagtactt caccgtgtat aacgagctga 2940

ccaaagtgaa atacgtgacc gagggaatga gaaagcccgc cttcctgagc ggcgagcaga 3000

aaaaggccat cgtggacctg ctgttcaaga ccaaccggaa agtgaccgtg aagcagctga 3060

aagaggacta cttcaagaaa atcgagtgct tcgactccgt ggaaatctcc ggcgtggaag 3120

atcggttcaa cgcctccctg ggcacatacc acgatctgct gaaaattatc aaggacaagg 3180

acttcctgga caatgaggaa aacgaggaca ttctggaaga tatcgtgctg accctgacac 3240

tgtttgagga cagagagatg atcgaggaac ggctgaaaac ctatgcccac ctgttcgacg 3300

acaaagtgat gaagcagctg aagcggcgga gatacaccgg ctggggcagg ctgagccgga 3360

agctgatcaa cggcatccgg gacaagcagt ccggcaagac aatcctggat ttcctgaagt 3420

ccgacggctt cgccaacaga aacttcatgc agctgatcca cgacgacagc ctgaccttta 3480

aagaggacat ccagaaagcc caggtgtccg gccagggcga tagcctgcac gagcacattg 3540

ccaatctggc cggcagcccc gccattaaga agggcatcct gcagacagtg aaggtggtgg 3600

acgagctcgt gaaagtgatg ggccggcaca agcccgagaa catcgtgatc gaaatggcca 3660

gagagaacca gaccacccag aagggacaga agaacagccg cgagagaatg aagcggatcg 3720

aagagggcat caaagagctg ggcagccaga tcctgaaaga acaccccgtg gaaaacaccc 3780

agctgcagaa cgagaagctg tacctgtact acctgcagaa tgggcgggat atgtacgtgg 3840

accaggaact ggacatcaac cggctgtccg actacgatgt ggaccatatc gtgcctcaga 3900

gctttctgaa ggacgactcc atcgacaaca aggtgctgac cagaagcgac aagaaccggg 3960

gcaagagcga caacgtgccc tccgaagagg tcgtgaagaa gatgaagaac tactggcggc 4020

agctgctgaa cgccaagctg attacccaga gaaagttcga caatctgacc aaggccgaga 4080

gaggcggcct gagcgaactg gataaggccg gcttcatcaa gagacagctg gtggaaaccc 4140

ggcagatcac aaagcacgtg gcacagatcc tggactcccg gatgaacact aagtacgacg 4200

agaatgacaa gctgatccgg gaagtgaaag tgatcaccct gaagtccaag ctggtgtccg 4260

atttccggaa ggatttccag ttttacaaag tgcgcgagat caacaactac caccacgccc 4320

acgacgccta cctgaacgcc gtcgtgggaa ccgccctgat caaaaagtac cctaagctgg 4380

aaagcgagtt cgtgtacggc gactacaagg tgtacgacgt gcggaagatg atcgccaaga 4440

gcgagcagga aatcggcaag gctaccgcca agtacttctt ctacagcaac atcatgaact 4500

ttttcaagac cgagattacc ctggccaacg gcgagatccg gaagcggcct ctgatcgaga 4560

caaacggcga aaccggggag atcgtgtggg ataagggccg ggattttgcc accgtgcgga 4620

aagtgctgag catgccccaa gtgaatatcg tgaaaaagac cgaggtgcag acaggcggct 4680

tcagcaaaga gtctatcctg cccaagagga acagcgataa gctgatcgcc agaaagaagg 4740

actgggaccc taagaagtac ggcggcttcg acagccccac cgtggcctat tctgtgctgg 4800

tggtggccaa agtggaaaag ggcaagtcca agaaactgaa gagtgtgaaa gagctgctgg 4860

ggatcaccat catggaaaga agcagcttcg agaagaatcc catcgacttt ctggaagcca 4920

agggctacaa agaagtgaaa aaggacctga tcatcaagct gcctaagtac tccctgttcg 4980

agctggaaaa cggccggaag agaatgctgg cctctgccgg cgaactgcag aagggaaacg 5040

aactggccct gccctccaaa tatgtgaact tcctgtacct ggccagccac tatgagaagc 5100

tgaagggctc ccccgaggat aatgagcaga aacagctgtt tgtggaacag cacaagcact 5160

acctggacga gatcatcgag cagatcagcg agttctccaa gagagtgatc ctggccgacg 5220

ctaatctgga caaagtgctg tccgcctaca acaagcaccg ggataagccc atcagagagc 5280

aggccgagaa tatcatccac ctgtttaccc tgaccaatct gggagcccct gccgccttca 5340

agtactttga caccaccatc gaccggaaga ggtacaccag caccaaagag gtgctggacg 5400

ccaccctgat ccaccagagc atcaccggcc tgtacgagac acggatcgac ctgtctcagc 5460

tgggaggcga cccgagccct ggtggcctcg tggctcctgc gttcacccgg agcagcagcc 5520

gcaagcgcgc ccggccccca gccgaacccg ggagtgacca gcccgcgccg ctcgcgcgcc 5580

ggaggttacg gctgcctgga ttggactcct gccccagttc tctgcctgag cccagttccc 5640

cagctgagcc cagccctcca gctgacccta gccctccagc tgaccctggc tcccccgttt 5700

gcccatcccc cgtcaagcga acaaagagta caactgttta tgttggtcaa cagccgggca 5760

agatcccctc agaggactcc gtcgaattca gccccaagaa gaagagaaag gtggaggcca 5820

gctaagaatt cctagagctc gctgatcagc ctcgactgtg ccttctagtt gccagccatc 5880

tgttgtttgc ccctcccccg tgccttcctt gaccctggaa ggtgccactc ccactgtcct 5940

ttcctaataa aatgaggaaa ttgcatcgca ttgtctgagt aggtgtcatt ctattctggg 6000

gggtggggtg gggcaggaca gcaaggggga ggattgggaa gagaatagca ggcatgcaat 6060

tccctgggga gcggccgcag gaacccctag tgatggagtt ggccactccc tctctgcgcg 6120

ctcgctcgct cactgaggcc gggcgaccaa aggtcgcccg acgcccgggc tttgcccggg 6180

cggcctcagt gagcgagcga gcgcgcagct gcctgcaggg gcgcctgatg cggtattttc 6240

tccttacgca tctgtgcggt atttcacacc gcatacgtca aagcaaccat agtacgcgcc 6300

ctgtagcggc gcattaagcg cggcgggtgt ggtggttacg cgcagcgtga ccgctacact 6360

tgccagcgcc ctagcgcccg ctcctttcgc tttcttccct tcctttctcg ccacgttcgc 6420

cggctttccc cgtcaagctc taaatcgggg gctcccttta gggttccgat ttagtgcttt 6480

acggcacctc gaccccaaaa aacttgattt gggtgatggt tcacgtagtg ggccatcgcc 6540

ctgatagacg gtttttcgcc ctttgacgtt ggagtccacg ttctttaata gtggactctt 6600

gttccaaact ggaacaacac tcaaccctat ctcgggctat tcttttgatt tataagggat 6660

tttgccgatt tcggcctatt ggttaaaaaa tgagctgatt taacaaaaat ttaacgcgaa 6720

ttttaacaaa atattaacgt ttacaatttt atggtgcact ctcagtacaa tctgctctga 6780

tgccgcatag ttaagccagc cccgacaccc gccaacaccc gctgacgcgc cctgacgggc 6840

ttgtctgctc ccggcatccg cttacagaca agctgtgacc gtctccggga gctgcatgtg 6900

tcagaggttt tcaccgtcat caccgaaacg cgcgagacga aagggcctcg tgatacgcct 6960

atttttatag gttaatgtca tgataataat ggtttcttag acgtcaggtg gcacttttcg 7020

gggaaatgtg cgcggaaccc ctatttgttt atttttctaa atacattcaa atatgtatcc 7080

gctcatgaga caataaccct gataaatgct tcaataatat tgaaaaagga agagtatgag 7140

tattcaacat ttccgtgtcg cccttattcc cttttttgcg gcattttgcc ttcctgtttt 7200

tgctcaccca gaaacgctgg tgaaagtaaa agatgctgaa gatcagttgg gtgcacgagt 7260

gggttacatc gaactggatc tcaacagcgg taagatcctt gagagttttc gccccgaaga 7320

acgttttcca atgatgagca cttttaaagt tctgctatgt ggcgcggtat tatcccgtat 7380

tgacgccggg caagagcaac tcggtcgccg catacactat tctcagaatg acttggttga 7440

gtactcacca gtcacagaaa agcatcttac ggatggcatg acagtaagag aattatgcag 7500

tgctgccata accatgagtg ataacactgc ggccaactta cttctgacaa cgatcggagg 7560

accgaaggag ctaaccgctt ttttgcacaa catgggggat catgtaactc gccttgatcg 7620

ttgggaaccg gagctgaatg aagccatacc aaacgacgag cgtgacacca cgatgcctgt 7680

agcaatggca acaacgttgc gcaaactatt aactggcgaa ctacttactc tagcttcccg 7740

gcaacaatta atagactgga tggaggcgga taaagttgca ggaccacttc tgcgctcggc 7800

ccttccggct ggctggttta ttgctgataa atctggagcc ggtgagcgtg gaagccgcgg 7860

tatcattgca gcactggggc cagatggtaa gccctcccgt atcgtagtta tctacacgac 7920

ggggagtcag gcaactatgg atgaacgaaa tagacagatc gctgagatag gtgcctcact 7980

gattaagcat tggtaactgt cagaccaagt ttactcatat atactttaga ttgatttaaa 8040

acttcatttt taatttaaaa ggatctaggt gaagatcctt tttgataatc tcatgaccaa 8100

aatcccttaa cgtgagtttt cgttccactg agcgtcagac cccgtagaaa agatcaaagg 8160

atcttcttga gatccttttt ttctgcgcgt aatctgctgc ttgcaaacaa aaaaaccacc 8220

gctaccagcg gtggtttgtt tgccggatca agagctacca actctttttc cgaaggtaac 8280

tggcttcagc agagcgcaga taccaaatac tgtccttcta gtgtagccgt agttaggcca 8340

ccacttcaag aactctgtag caccgcctac atacctcgct ctgctaatcc tgttaccagt 8400

ggctgctgcc agtggcgata agtcgtgtct taccgggttg gactcaagac gatagttacc 8460

ggataaggcg cagcggtcgg gctgaacggg gggttcgtgc acacagccca gcttggagcg 8520

aacgacctac accgaactga gatacctaca gcgtgagcta tgagaaagcg ccacgcttcc 8580

cgaagggaga aaggcggaca ggtatccggt aagcggcagg gtcggaacag gagagcgcac 8640

gagggagctt ccagggggaa acgcctggta tctttatagt cctgtcgggt ttcgccacct 8700

ctgacttgag cgtcgatttt tgtgatgctc gtcagggggg cggagcctat ggaaaaacgc 8760

cagcaacgcg gcctttttac ggttcctggc cttttgctgg ccttttgctc acatgt 8816

<210> 7
<211> 28
<212> DNA
<213> Mus musculus

<400> 7
tcaagacaaa cctgagcact tttcatca 28

<210> 8
<211> 28
<212> DNA
<213> Mus musculus

<400> 8
acccaatgtg gttaaaggaa agaaccaa 28

<210> 9
<211> 23
<212> DNA
<213> Mus musculus

<400> 9
cctgtgttaa tctactatgg gct 23

<210> 10
<211> 23
<212> DNA
<213> Mus musculus

<400> 10
cctagacggg atctatctca ctt 23

<210> 11
<211> 20
<212> DNA
<213> Mus musculus

<400> 11
tggatctacc ccatttctgc 20

<210> 12
<211> 20
<212> DNA
<213> Mus musculus

<400> 12
aaattttggt ggcattgcat 20

<210> 13
<211> 20
<212> DNA
<213> Mus musculus

<400>  13
acagggggac attgcaaata                                                    20


<210>  14
<211>  20
<212>  DNA
<213>  Mus musculus

<400>  14
ccacacaggt aaaggggcta                                                    20


<210>  15
<211>  23
<212>  DNA
<213>  Mus musculus

<400>  15
cctcaatgag tagtttgttt ccc                                                23


<210>  16
<211>  23
<212>  DNA
<213>  Mus musculus

<400>  16
tgtgtccata tagtactagg agg                                                23


<210>  17
<211>  23
<212>  DNA
<213>  Mus musculus

<400>  17
ccagtctttc tagaagatgg gcg                                                23


<210>  18
<211>  30
<212>  DNA
<213>  Mus musculus

<400>  18
ctcagagagc ctcggctagg taggggatcg                                         30


<210>  19
<211>  30
<212>  DNA
<213>  Artificial Sequence

<220>
<223>  SYNTHESIZED ORIGONUCLEOTIDE

<400>  19
acgtcaatgg aaagtcccta ttggcgttac                                         30


<210>  20

<211>  28
<212>  DNA
<213>  Artificial Sequence

<220>
<223>  SYNTHESIZED ORIGONUCLEOTIDE

<400>  20
tgccatgaac aaaggttggc tataaaga                                          28


<210>  21
<211>  28
<212>  DNA
<213>  Mus musculus

<400>  21
gcctgccaag taactactct tgtgtgct                                          28


<210>  22
<211>  28
<212>  DNA
<213>  Mus musculus

<400>  22
aatttgatgg gggaaaactt gaatgaaa                                          28


<210>  23
<211>  23
<212>  DNA
<213>  Mus musculus

<400>  23
gtatccagga acatcttgag tgg                                               23


<210>  24
<211>  23
<212>  DNA
<213>  Mus musculus

<400>  24
cccggtcagg ctctagatag tca                                               23


<210>  25
<211>  28
<212>  DNA
<213>  Mus musculus

<400>  25
cttgtactca aaaccttctg ccccaact                                          28


<210>  26
<211>  28
<212>  DNA
<213>  Mus musculus

<400>  26
acttctgtcc tctcaggggc actctact                                          28

<210>  27
<211>  69
<212>  DNA
<213>  Artificial Sequence

<220>
<223>  DERIVED FROM BACTERIOPHAGE P1

<400>  27
gtatccagga acatcttaaa cataacttcg tatagcatac attatacgaa gttatggcgc        60

gccgagtgg                                                                69


<210>  28
<211>  85
<212>  DNA
<213>  Artificial Sequence

<220>
<223>  DERIVED FROM BACTERIOPHAGE P1

<400>  28
cccggtcgcg ccaattcgat atcaagctat aacttcgtat agcatacatt atacgaagtt        60

atgcggccca ggctctagat agtca                                              85


<210>  29
<211>  52
<212>  PRT
<213>  Mus musculus

<400>  29

Pro Ser Pro Gly Gly Leu Val Ala Pro Ala Phe Thr Arg Ser Ser Ser
1               5                   10                  15


Arg Lys Arg Ala Arg Pro Pro Ala Glu Pro Gly Ser Asp Gln Pro Ala
            20                  25                  30


Pro Leu Ala Arg Arg Arg Leu Arg Leu Pro Gly Leu Asp Ser Cys Pro
        35                  40                  45


Ser Ser Leu Pro
    50


<210>  30
<211>  8756
<212>  DNA
<213>  Artificial Sequence

<220>
<223>  pX330-pFmC VECTOR DERIVED FROM STREPTOCOCCUS PYOGENES AND MUS
        MUSCLUS

<400>  30

gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccatcca cggagtccca gcagccgaca agaagtacag catcggcctg gacatcggca 1380

ccaactctgt gggctgggcc gtgatcaccg acgagtacaa ggtgcccagc aagaaattca 1440

aggtgctggg caacaccgac cggcacagca tcaagaagaa cctgatcgga gccctgctgt 1500

tcgacagcgg cgaaacagcc gaggccaccc ggctgaagag aaccgccaga agaagataca 1560

ccagacggaa gaaccggatc tgctatctgc aagagatctt cagcaacgag atggccaagg 1620

tggacgacag cttcttccac agactggaag agtccttcct ggtggaagag gataagaagc 1680

acgagcggca ccccatcttc ggcaacatcg tggacgaggt ggcctaccac gagaagtacc 1740

ccaccatcta ccacctgaga aagaaactgg tggacagcac cgacaaggcc gacctgcggc 1800

tgatctatct ggccctggcc cacatgatca agttccgggg ccacttcctg atcgagggcg 1860

acctgaaccc cgacaacagc gacgtggaca agctgttcat ccagctggtg cagacctaca 1920

accagctgtt cgaggaaaac cccatcaacg ccagcggcgt ggacgccaag gccatcctgt 1980

ctgccagact gagcaagagc agacggctgg aaaatctgat cgcccagctg cccggcgaga 2040

agaagaatgg cctgttcgga aacctgattg ccctgagcct gggcctgacc cccaacttca 2100

agagcaactt cgacctggcc gaggatgcca aactgcagct gagcaaggac acctacgacg 2160

acgacctgga caacctgctg gcccagatcg gcgaccagta cgccgacctg tttctggccg 2220

ccaagaacct gtccgacgcc atcctgctga gcgacatcct gagagtgaac accgagatca 2280

ccaaggcccc cctgagcgcc tctatgatca agagatacga cgagcaccac caggacctga 2340

ccctgctgaa agctctcgtg cggcagcagc tgcctgagaa gtacaaagag attttcttcg 2400

accagagcaa gaacggctac gccggctaca ttgacggcgg agccagccag gaagagttct 2460

acaagttcat caagcccatc ctggaaaaga tggacggcac cgaggaactg ctcgtgaagc 2520

tgaacagaga ggacctgctg cggaagcagc ggaccttcga caacggcagc atcccccacc 2580

agatccacct gggagagctg cacgccattc tgcggcggca ggaagatttt tacccattcc 2640

tgaaggacaa ccgggaaaag atcgagaaga tcctgacctt ccgcatcccc tactacgtgg 2700

gccctctggc caggggaaac agcagattcg cctggatgac cagaaagagc gaggaaacca 2760

tcaccccctg gaacttcgag gaagtggtgg acaagggcgc ttccgcccag agcttcatcg 2820

agcggatgac caacttcgat aagaacctgc ccaacgagaa ggtgctgccc aagcacagcc 2880

tgctgtacga gtacttcacc gtgtataacg agctgaccaa agtgaaatac gtgaccgagg 2940

gaatgagaaa gcccgccttc ctgagcggcg agcagaaaaa ggccatcgtg gacctgctgt 3000

tcaagaccaa ccggaaagtg accgtgaagc agctgaaaga ggactacttc aagaaaatcg 3060

agtgcttcga ctccgtggaa atctccggcg tggaagatcg gttcaacgcc tccctgggca 3120

cataccacga tctgctgaaa attatcaagg acaaggactt cctggacaat gaggaaaacg 3180

aggacattct ggaagatatc gtgctgaccc tgacactgtt tgaggacaga gagatgatcg 3240

aggaacggct gaaaacctat gcccacctgt tcgacgacaa agtgatgaag cagctgaagc 3300

ggcggagata caccggctgg ggcaggctga gccggaagct gatcaacggc atccgggaca 3360

agcagtccgg caagacaatc ctggatttcc tgaagtccga cggcttcgcc aacagaaact 3420

tcatgcagct gatccacgac gacagcctga cctttaaaga ggacatccag aaagcccagg 3480

tgtccggcca gggcgatagc ctgcacgagc acattgccaa tctggccggc agccccgcca 3540

ttaagaaggg catcctgcag acagtgaagg tggtggacga gctcgtgaaa gtgatgggcc 3600

ggcacaagcc cgagaacatc gtgatcgaaa tggccagaga gaaccagacc acccagaagg 3660

gacagaagaa cagccgcgag agaatgaagc ggatcgaaga gggcatcaaa gagctgggca 3720

gccagatcct gaaagaacac cccgtggaaa acacccagct gcagaacgag aagctgtacc 3780

tgtactacct gcagaatggg cgggatatgt acgtggacca ggaactggac atcaaccggc 3840

tgtccgacta cgatgtggac catatcgtgc ctcagagctt tctgaaggac gactccatcg 3900

acaacaaggt gctgaccaga agcgacaaga accggggcaa gagcgacaac gtgccctccg 3960

aagaggtcgt gaagaagatg aagaactact ggcggcagct gctgaacgcc aagctgatta 4020

cccagagaaa gttcgacaat ctgaccaagg ccgagagagg cggcctgagc gaactggata 4080

aggccggctt catcaagaga cagctggtgg aaacccggca gatcacaaag cacgtggcac 4140

agatcctgga ctcccggatg aacactaagt acgacgagaa tgacaagctg atccgggaag 4200

tgaaagtgat caccctgaag tccaagctgg tgtccgattt ccggaaggat ttccagtttt 4260

acaaagtgcg cgagatcaac aactaccacc acgcccacga cgcctacctg aacgccgtcg 4320

tgggaaccgc cctgatcaaa aagtacccta agctggaaag cgagttcgtg tacggcgact 4380

acaaggtgta cgacgtgcgg aagatgatcg ccaagagcga gcaggaaatc ggcaaggcta 4440

ccgccaagta cttcttctac agcaacatca tgaacttttt caagaccgag attaccctgg 4500

ccaacggcga gatccggaag cggcctctga tcgagacaaa cggcgaaacc ggggagatcg 4560

tgtgggataa gggccgggat tttgccaccg tgcggaaagt gctgagcatg ccccaagtga 4620

atatcgtgaa aaagaccgag gtgcagacag gcggcttcag caaagagtct atcctgccca 4680

agaggaacag cgataagctg atcgccagaa agaaggactg ggaccctaag aagtacggcg 4740

gcttcgacag ccccaccgtg gcctattctg tgctggtggt ggccaaagtg gaaaagggca 4800

agtccaagaa actgaagagt gtgaaagagc tgctggggat caccatcatg gaaagaagca 4860

gcttcgagaa gaatcccatc gactttctgg aagccaaggg ctacaaagaa gtgaaaaagg 4920

acctgatcat caagctgcct aagtactccc tgttcgagct ggaaaacggc cggaagagaa 4980

tgctggcctc tgccggcgaa ctgcagaagg gaaacgaact ggccctgccc tccaaatatg 5040

tgaacttcct gtacctggcc agccactatg agaagctgaa gggctccccc gaggataatg 5100

agcagaaaca gctgtttgtg gaacagcaca agcactacct ggacgagatc atcgagcaga 5160

tcagcgagtt ctccaagaga gtgatcctgg ccgacgctaa tctggacaaa gtgctgtccg 5220

cctacaacaa gcaccgggat aagcccatca gagagcaggc cgagaatatc atccacctgt 5280

ttaccctgac caatctggga gcccctgccg ccttcaagta ctttgacacc accatcgacc 5340

ggaagaggta caccagcacc aaagaggtgc tggacgccac cctgatccac cagagcatca 5400

ccggcctgta cgagacacgg atcgacctgt ctcagctggg aggcgacccg agccctggtg 5460

gcctcgtggc tcctgcgttc acccggagca gcagccgcaa gcgcgcccgg cccccagccg 5520

aacccggagg tgaccagccc gcgccgctcg cgcgccggag gttacggctg cctggattgg 5580

actcctgccc cagttctctg cctgagccca gttccccagc tgagcccagc cctccagctg 5640

accctagccc tccagctgac cctggctccc ccgtttgccc atcccccgtc aagcgaacaa 5700

agagtacaac tgtttatgtt ggtcaacagc cgggcaagat cccctcagag gactccgtcg 5760

aattctaatt cctagagctc gctgatcagc ctcgactgtg ccttctagtt gccagccatc 5820

tgttgtttgc ccctcccccg tgccttcctt gaccctggaa ggtgccactc ccactgtcct 5880

ttcctaataa aatgaggaaa ttgcatcgca ttgtctgagt aggtgtcatt ctattctggg 5940

gggtggggtg gggcaggaca gcaaggggga ggattgggaa gagaatagca ggcatgcaat 6000

tccctgggga gcggccgcag gaacccctag tgatggagtt ggccactccc tctctgcgcg 6060

ctcgctcgct cactgaggcc gggcgaccaa aggtcgcccg acgcccgggc tttgcccggg 6120

cggcctcagt gagcgagcga gcgcgcagct gcctgcaggg gcgcctgatg cggtattttc 6180

tccttacgca tctgtgcggt atttcacacc gcatacgtca aagcaaccat agtacgcgcc 6240

ctgtagcggc gcattaagcg cggcgggtgt ggtggttacg cgcagcgtga ccgctacact 6300

tgccagcgcc ctagcgcccg ctcctttcgc tttcttccct tcctttctcg ccacgttcgc 6360

cggctttccc cgtcaagctc taaatcgggg gctcccttta gggttccgat ttagtgcttt 6420

acggcacctc gaccccaaaa aacttgattt gggtgatggt tcacgtagtg ggccatcgcc 6480

ctgatagacg gtttttcgcc ctttgacgtt ggagtccacg ttctttaata gtggactctt 6540

gttccaaact ggaacaacac tcaaccctat ctcgggctat tcttttgatt tataagggat 6600

tttgccgatt tcggcctatt ggttaaaaaa tgagctgatt taacaaaaat ttaacgcgaa 6660

ttttaacaaa atattaacgt ttacaatttt atggtgcact ctcagtacaa tctgctctga 6720

tgccgcatag ttaagccagc cccgacaccc gccaacaccc gctgacgcgc cctgacgggc 6780

ttgtctgctc ccggcatccg cttacagaca agctgtgacc gtctccggga gctgcatgtg 6840

tcagaggttt tcaccgtcat caccgaaacg cgcgagacga aagggcctcg tgatacgcct 6900

atttttatag gttaatgtca tgataataat ggtttcttag acgtcaggtg gcacttttcg 6960

gggaaatgtg cgcggaaccc ctatttgttt atttttctaa atacattcaa atatgtatcc 7020

gctcatgaga caataaccct gataaatgct tcaataatat tgaaaaagga agagtatgag 7080

tattcaacat ttccgtgtcg cccttattcc cttttttgcg gcattttgcc ttcctgtttt 7140

tgctcaccca gaaacgctgg tgaaagtaaa agatgctgaa gatcagttgg gtgcacgagt 7200

gggttacatc gaactggatc tcaacagcgg taagatcctt gagagttttc gccccgaaga 7260

acgttttcca atgatgagca cttttaaagt tctgctatgt ggcgcggtat tatcccgtat 7320

tgacgccggg caagagcaac tcggtcgccg catacactat tctcagaatg acttggttga 7380

gtactcacca gtcacagaaa agcatcttac ggatggcatg acagtaagag aattatgcag 7440

tgctgccata accatgagtg ataacactgc ggccaactta cttctgacaa cgatcggagg 7500

accgaaggag ctaaccgctt ttttgcacaa catgggggat catgtaactc gccttgatcg 7560

ttgggaaccg gagctgaatg aagccatacc aaacgacgag cgtgacacca cgatgcctgt 7620

agcaatggca acaacgttgc gcaaactatt aactggcgaa ctacttactc tagcttcccg 7680

gcaacaatta atagactgga tggaggcgga taaagttgca ggaccacttc tgcgctcggc 7740

ccttccggct ggctggttta ttgctgataa atctggagcc ggtgagcgtg gaagccgcgg 7800

tatcattgca gcactggggc cagatggtaa gccctcccgt atcgtagtta tctacacgac 7860

ggggagtcag gcaactatgg atgaacgaaa tagacagatc gctgagatag gtgcctcact 7920

gattaagcat tggtaactgt cagaccaagt ttactcatat atactttaga ttgatttaaa 7980

acttcatttt taatttaaaa ggatctaggt gaagatcctt tttgataatc tcatgaccaa 8040

aatcccttaa cgtgagtttt cgttccactg agcgtcagac cccgtagaaa agatcaaagg 8100

atcttcttga gatccttttt ttctgcgcgt aatctgctgc ttgcaaacaa aaaaaccacc 8160

gctaccagcg gtggtttgtt tgccggatca agagctacca actctttttc cgaaggtaac 8220

tggcttcagc agagcgcaga taccaaatac tgtccttcta gtgtagccgt agttaggcca 8280

ccacttcaag aactctgtag caccgcctac atacctcgct ctgctaatcc tgttaccagt 8340

ggctgctgcc agtggcgata agtcgtgtct taccgggttg gactcaagac gatagttacc 8400

ggataaggcg cagcggtcgg gctgaacggg gggttcgtgc acacagccca gcttggagcg 8460

aacgacctac accgaactga gatacctaca gcgtgagcta tgagaaagcg ccacgcttcc 8520

cgaagggaga aaggcggaca ggtatccggt aagcggcagg gtcggaacag gagagcgcac 8580

gagggagctt ccagggggaa acgcctggta tctttatagt cctgtcgggt ttcgccacct 8640

ctgacttgag cgtcgatttt tgtgatgctc gtcagggggg cggagcctat ggaaaaacgc 8700

cagcaacgcg gcctttttac ggttcctggc cttttgctgg ccttttgctc acatgt 8756

<210> 31
<211> 8600
<212> DNA
<213> Artificial Sequence

<220>
<223> pX330-pCmC VECTOR DERIVED FROM STREPTOCOCCUS PYOGENES AND MUS
      MUSCLUS

<400> 31
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccatcca cggagtccca gcagccgaca agaagtacag catcggcctg gacatcggca 1380

ccaactctgt gggctgggcc gtgatcaccg acgagtacaa ggtgcccagc aagaaattca 1440

aggtgctggg caacaccgac cggcacagca tcaagaagaa cctgatcgga gccctgctgt 1500

tcgacagcgg cgaaacagcc gaggccaccc ggctgaagag aaccgccaga agaagataca 1560

ccagacggaa gaaccggatc tgctatctgc aagagatctt cagcaacgag atggccaagg 1620

tggacgacag cttcttccac agactggaag agtccttcct ggtggaagag gataagaagc 1680

acgagcggca ccccatcttc ggcaacatcg tggacgaggt ggcctaccac gagaagtacc 1740

ccaccatcta ccacctgaga aagaaactgg tggacagcac cgacaaggcc gacctgcggc 1800

tgatctatct ggccctggcc cacatgatca agttccgggg ccacttcctg atcgagggcg 1860

acctgaaccc cgacaacagc gacgtggaca agctgttcat ccagctggtg cagacctaca 1920

accagctgtt cgaggaaaac cccatcaacg ccagcggcgt ggacgccaag gccatcctgt 1980

ctgccagact gagcaagagc agacggctgg aaaatctgat cgcccagctg cccggcgaga 2040

agaagaatgg cctgttcgga aacctgattg ccctgagcct gggcctgacc cccaacttca 2100

agagcaactt cgacctggcc gaggatgcca aactgcagct gagcaaggac acctacgacg 2160

acgacctgga caacctgctg gcccagatcg gcgaccagta cgccgacctg tttctggccg 2220

ccaagaacct gtccgacgcc atcctgctga gcgacatcct gagagtgaac accgagatca 2280

ccaaggcccc cctgagcgcc tctatgatca agagatacga cgagcaccac caggacctga 2340

ccctgctgaa agctctcgtg cggcagcagc tgcctgagaa gtacaaagag attttcttcg 2400

accagagcaa gaacggctac gccggctaca ttgacggcgg agccagccag gaagagttct 2460

acaagttcat caagcccatc ctggaaaaga tggacggcac cgaggaactg ctcgtgaagc 2520

tgaacagaga ggacctgctg cggaagcagc ggaccttcga caacggcagc atcccccacc 2580

agatccacct gggagagctg cacgccattc tgcggcggca ggaagatttt tacccattcc 2640

tgaaggacaa ccgggaaaag atcgagaaga tcctgacctt ccgcatcccc tactacgtgg 2700

gccctctggc caggggaaac agcagattcg cctggatgac cagaaagagc gaggaaacca 2760

tcaccccctg gaacttcgag gaagtggtgg acaagggcgc ttccgcccag agcttcatcg 2820

agcggatgac caacttcgat aagaacctgc ccaacgagaa ggtgctgccc aagcacagcc 2880

tgctgtacga gtacttcacc gtgtataacg agctgaccaa agtgaaatac gtgaccgagg 2940

gaatgagaaa gcccgccttc ctgagcggcg agcagaaaaa ggccatcgtg gacctgctgt 3000

tcaagaccaa ccggaaagtg accgtgaagc agctgaaaga ggactacttc aagaaaatcg 3060

agtgcttcga ctccgtggaa atctccggcg tggaagatcg gttcaacgcc tccctgggca 3120

cataccacga tctgctgaaa attatcaagg acaaggactt cctggacaat gaggaaaacg 3180

aggacattct ggaagatatc gtgctgaccc tgacactgtt tgaggacaga gagatgatcg 3240

aggaacggct gaaaacctat gcccacctgt tcgacgacaa agtgatgaag cagctgaagc 3300

ggcggagata caccggctgg ggcaggctga gccggaagct gatcaacggc atccgggaca 3360

agcagtccgg caagacaatc ctggatttcc tgaagtccga cggcttcgcc aacagaaact 3420

tcatgcagct gatccacgac gacagcctga cctttaaaga ggacatccag aaagcccagg 3480

tgtccggcca gggcgatagc ctgcacgagc acattgccaa tctggccggc agccccgcca 3540

ttaagaaggg catcctgcag acagtgaagg tggtggacga gctcgtgaaa gtgatgggcc 3600

ggcacaagcc cgagaacatc gtgatcgaaa tggccagaga gaaccagacc acccagaagg 3660

gacagaagaa cagccgcgag agaatgaagc ggatcgaaga gggcatcaaa gagctgggca 3720

gccagatcct gaaagaacac cccgtggaaa acacccagct gcagaacgag aagctgtacc 3780

tgtactacct gcagaatggg cgggatatgt acgtggacca ggaactggac atcaaccggc 3840

tgtccgacta cgatgtggac catatcgtgc ctcagagctt tctgaaggac gactccatcg 3900

acaacaaggt gctgaccaga agcgacaaga accggggcaa gagcgacaac gtgccctccg 3960

aagaggtcgt gaagaagatg aagaactact ggcggcagct gctgaacgcc aagctgatta 4020

cccagagaaa gttcgacaat ctgaccaagg ccgagagagg cggcctgagc gaactggata 4080

aggccggctt catcaagaga cagctggtgg aaacccggca gatcacaaag cacgtggcac 4140

agatcctgga ctcccggatg aacactaagt acgacgagaa tgacaagctg atccgggaag 4200

tgaaagtgat caccctgaag tccaagctgg tgtccgattt ccggaaggat ttccagtttt 4260

acaaagtgcg cgagatcaac aactaccacc acgcccacga cgcctacctg aacgccgtcg 4320

tgggaaccgc cctgatcaaa aagtacccta agctggaaag cgagttcgtg tacggcgact 4380

acaaggtgta cgacgtgcgg aagatgatcg ccaagagcga gcaggaaatc ggcaaggcta 4440

ccgccaagta cttcttctac agcaacatca tgaacttttt caagaccgag attaccctgg 4500

ccaacggcga gatccggaag cggcctctga tcgagacaaa cggcgaaacc ggggagatcg 4560

tgtgggataa gggccgggat tttgccaccg tgcggaaagt gctgagcatg ccccaagtga 4620

atatcgtgaa aaagaccgag gtgcagacag gcggcttcag caaagagtct atcctgccca 4680

agaggaacag cgataagctg atcgccagaa agaaggactg ggaccctaag aagtacggcg 4740

gcttcgacag ccccaccgtg gcctattctg tgctggtggt ggccaaagtg gaaaagggca 4800

agtccaagaa actgaagagt gtgaaagagc tgctggggat caccatcatg gaaagaagca 4860

gcttcgagaa gaatcccatc gactttctgg aagccaaggg ctacaaagaa gtgaaaaagg 4920

acctgatcat caagctgcct aagtactccc tgttcgagct ggaaaacggc cggaagagaa 4980

tgctggcctc tgccggcgaa ctgcagaagg gaaacgaact ggccctgccc tccaaatatg 5040

tgaacttcct gtacctggcc agccactatg agaagctgaa gggctccccc gaggataatg 5100

agcagaaaca gctgtttgtg gaacagcaca agcactacct ggacgagatc atcgagcaga 5160

tcagcgagtt ctccaagaga gtgatcctgg ccgacgctaa tctggacaaa gtgctgtccg 5220

cctacaacaa gcaccgggat aagcccatca gagagcaggc cgagaatatc atccacctgt 5280

ttaccctgac caatctggga gcccctgccg ccttcaagta ctttgacacc accatcgacc 5340

ggaagaggta caccagcacc aaagaggtgc tggacgccac cctgatccac cagagcatca 5400

ccggcctgta cgagacacgg atcgacctgt ctcagctggg aggcgacgag cccagttccc 5460

cagctgagcc cagccctcca gctgacccta gccctccagc tgaccctggc tcccccgttt 5520

gcccatcccc cgtcaagcga acaaagagta caactgttta tgttggtcaa cagccgggca 5580

agatcccctc agaggactcc gtcgaattct aattcctaga gctcgctgat cagcctcgac 5640

tgtgccttct agttgccagc catctgttgt ttgcccctcc cccgtgcctt ccttgaccct 5700

ggaaggtgcc actcccactg tcctttccta ataaaatgag gaaattgcat cgcattgtct 5760

gagtaggtgt cattctattc tggggggtgg ggtggggcag gacagcaagg gggaggattg 5820

ggaagagaat agcaggcatg caattccctg gggagcggcc gcaggaaccc ctagtgatgg 5880

agttggccac tccctctctg cgcgctcgct cgctcactga ggccgggcga ccaaaggtcg 5940

cccgacgccc gggctttgcc cgggcggcct cagtgagcga gcgagcgcgc agctgcctgc 6000

aggggcgcct gatgcggtat tttctcctta cgcatctgtg cggtatttca caccgcatac 6060

gtcaaagcaa ccatagtacg cgccctgtag cggcgcatta agcgcggcgg gtgtggtggt 6120

tacgcgcagc gtgaccgcta cacttgccag cgccctagcg cccgctcctt tcgctttctt 6180

cccttccttt ctcgccacgt tcgccggctt tccccgtcaa gctctaaatc gggggctccc 6240

tttagggttc cgatttagtg ctttacggca cctcgacccc aaaaaacttg atttgggtga 6300

tggttcacgt agtgggccat cgccctgata gacggttttt cgccctttga cgttggagtc 6360

cacgttcttt aatagtggac tcttgttcca aactggaaca acactcaacc ctatctcggg 6420

ctattctttt gatttataag ggattttgcc gatttcggcc tattggttaa aaaatgagct 6480

gatttaacaa aaatttaacg cgaattttaa caaaatatta acgtttacaa ttttatggtg 6540

cactctcagt acaatctgct ctgatgccgc atagttaagc cagccccgac acccgccaac 6600

acccgctgac gcgccctgac gggcttgtct gctcccggca tccgcttaca gacaagctgt 6660

gaccgtctcc gggagctgca tgtgtcagag gttttcaccg tcatcaccga aacgcgcgag 6720

acgaaagggc ctcgtgatac gcctattttt ataggttaat gtcatgataa taatggtttc 6780

ttagacgtca ggtggcactt ttcggggaaa tgtgcgcgga acccctattt gtttattttt 6840

ctaaatacat tcaaatatgt atccgctcat gagacaataa ccctgataaa tgcttcaata 6900

atattgaaaa aggaagagta tgagtattca acatttccgt gtcgccctta ttcccttttt 6960

tgcggcattt tgccttcctg tttttgctca cccagaaacg ctggtgaaag taaaagatgc 7020

tgaagatcag ttgggtgcac gagtgggtta catcgaactg gatctcaaca gcggtaagat 7080

ccttgagagt tttcgccccg aagaacgttt tccaatgatg agcactttta aagttctgct 7140

atgtggcgcg gtattatccc gtattgacgc cgggcaagag caactcggtc gccgcataca 7200

ctattctcag aatgacttgg ttgagtactc accagtcaca gaaaagcatc ttacggatgg 7260

catgacagta agagaattat gcagtgctgc cataaccatg agtgataaca ctgcggccaa 7320

cttacttctg acaacgatcg gaggaccgaa ggagctaacc gcttttttgc acaacatggg 7380

ggatcatgta actcgccttg atcgttggga accggagctg aatgaagcca taccaaacga 7440

cgagcgtgac accacgatgc ctgtagcaat ggcaacaacg ttgcgcaaac tattaactgg 7500

cgaactactt actctagctt cccggcaaca attaatagac tggatggagg cggataaagt 7560

tgcaggacca cttctgcgct cggcccttcc ggctggctgg tttattgctg ataaatctgg 7620

agccggtgag cgtggaagcc gcggtatcat tgcagcactg gggccagatg gtaagccctc 7680

ccgtatcgta gttatctaca cgacggggag tcaggcaact atggatgaac gaaatagaca 7740

gatcgctgag ataggtgcct cactgattaa gcattggtaa ctgtcagacc aagtttactc 7800

atatatactt tagattgatt taaaacttca tttttaattt aaaaggatct aggtgaagat 7860

cctttttgat aatctcatga ccaaaatccc ttaacgtgag ttttcgttcc actgagcgtc 7920

agaccccgta gaaaagatca aaggatcttc ttgagatcct ttttttctgc gcgtaatctg 7980

ctgcttgcaa acaaaaaaac caccgctacc agcggtggtt tgtttgccgg atcaagagct 8040

accaactctt tttccgaagg taactggctt cagcagagcg cagataccaa atactgtcct 8100

tctagtgtag ccgtagttag gccaccactt caagaactct gtagcaccgc ctacatacct 8160

cgctctgcta atcctgttac cagtggctgc tgccagtggc gataagtcgt gtcttaccgg 8220

gttggactca agacgatagt taccggataa ggcgcagcgg tcgggctgaa cggggggttc 8280

gtgcacacag cccagcttgg agcgaacgac ctacaccgaa ctgagatacc tacagcgtga 8340

gctatgagaa agcgccacgc ttcccgaagg gagaaaggcg gacaggtatc cggtaagcgg 8400

cagggtcgga acaggagagc gcacgaggga gcttccaggg ggaaacgcct ggtatcttta 8460

tagtcctgtc gggtttcgcc acctctgact tgagcgtcga tttttgtgat gctcgtcagg 8520

ggggcggagc ctatggaaaa acgccagcaa cgcggccttt ttacggttcc tggccttttg 8580

ctggcctttt gctcacatgt 8600

<210> 32
<211> 8600
<212> DNA
<213> Artificial Sequence

<220>
<223> pX330-pMmC VECTOR DERIVED FROM STREPTOCOCCUS PYOGENES AND MUS
      MUSCLUS

<400> 32
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccatcca cggagtccca gcagccgaca agaagtacag catcggcctg gacatcggca 1380

ccaactctgt gggctgggcc gtgatcaccg acgagtacaa ggtgcccagc aagaaattca 1440

aggtgctggg caacaccgac cggcacagca tcaagaagaa cctgatcgga gccctgctgt 1500

tcgacagcgg cgaaacagcc gaggccaccc ggctgaagag aaccgccaga agaagataca 1560

ccagacggaa gaaccggatc tgctatctgc aagagatctt cagcaacgag atggccaagg 1620

tggacgacag cttcttccac agactggaag agtccttcct ggtggaagag gataagaagc 1680

acgagcggca ccccatcttc ggcaacatcg tggacgaggt ggcctaccac gagaagtacc 1740

ccaccatcta ccacctgaga aagaaactgg tggacagcac cgacaaggcc gacctgcggc 1800

tgatctatct ggccctggcc cacatgatca agttccgggg ccacttcctg atcgagggcg 1860

acctgaaccc cgacaacagc gacgtggaca agctgttcat ccagctggtg cagacctaca 1920

accagctgtt cgaggaaaac cccatcaacg ccagcggcgt ggacgccaag gccatcctgt 1980

ctgccagact gagcaagagc agacggctgg aaaatctgat cgcccagctg cccggcgaga 2040

agaagaatgg cctgttcgga aacctgattg ccctgagcct gggcctgacc cccaacttca 2100

agagcaactt cgacctggcc gaggatgcca aactgcagct gagcaaggac acctacgacg 2160

acgacctgga caacctgctg gcccagatcg gcgaccagta cgccgacctg tttctggccg 2220

ccaagaacct gtccgacgcc atcctgctga gcgacatcct gagagtgaac accgagatca 2280

ccaaggcccc cctgagcgcc tctatgatca agagatacga cgagcaccac caggacctga 2340

ccctgctgaa agctctcgtg cggcagcagc tgcctgagaa gtacaaagag attttcttcg 2400

accagagcaa gaacggctac gccggctaca ttgacggcgg agccagccag gaagagttct 2460

acaagttcat caagcccatc ctggaaaaga tggacggcac cgaggaactg ctcgtgaagc 2520

tgaacagaga ggacctgctg cggaagcagc ggaccttcga caacggcagc atcccccacc 2580

agatccacct gggagagctg cacgccattc tgcggcggca ggaagatttt tacccattcc 2640

tgaaggacaa ccgggaaaag atcgagaaga tcctgacctt ccgcatcccc tactacgtgg 2700

gccctctggc caggggaaac agcagattcg cctggatgac cagaaagagc gaggaaacca 2760

tcaccccctg gaacttcgag gaagtggtgg acaagggcgc ttccgcccag agcttcatcg 2820

agcggatgac caacttcgat aagaacctgc ccaacgagaa ggtgctgccc aagcacagcc 2880

tgctgtacga gtacttcacc gtgtataacg agctgaccaa agtgaaatac gtgaccgagg 2940

gaatgagaaa gcccgccttc ctgagcggcg agcagaaaaa ggccatcgtg gacctgctgt 3000

tcaagaccaa ccggaaagtg accgtgaagc agctgaaaga ggactacttc aagaaaatcg 3060

agtgcttcga ctccgtggaa atctccggcg tggaagatcg gttcaacgcc tccctgggca 3120

cataccacga tctgctgaaa attatcaagg acaaggactt cctggacaat gaggaaaacg 3180

aggacattct ggaagatatc gtgctgaccc tgacactgtt tgaggacaga gagatgatcg 3240

aggaacggct gaaaacctat gcccacctgt tcgacgacaa agtgatgaag cagctgaagc 3300

ggcggagata caccggctgg ggcaggctga gccggaagct gatcaacggc atccgggaca 3360

agcagtccgg caagacaatc ctggatttcc tgaagtccga cggcttcgcc aacagaaact 3420

tcatgcagct gatccacgac gacagcctga cctttaaaga ggacatccag aaagcccagg 3480

tgtccggcca gggcgatagc ctgcacgagc acattgccaa tctggccggc agccccgcca 3540

ttaagaaggg catcctgcag acagtgaagg tggtggacga gctcgtgaaa gtgatgggcc 3600

ggcacaagcc cgagaacatc gtgatcgaaa tggccagaga gaaccagacc acccagaagg 3660

gacagaagaa cagccgcgag agaatgaagc ggatcgaaga gggcatcaaa gagctgggca 3720

gccagatcct gaaagaacac cccgtggaaa acacccagct gcagaacgag aagctgtacc 3780

tgtactacct gcagaatggg cgggatatgt acgtggacca ggaactggac atcaaccggc 3840

tgtccgacta cgatgtggac catatcgtgc ctcagagctt tctgaaggac gactccatcg 3900

acaacaaggt gctgaccaga agcgacaaga accggggcaa gagcgacaac gtgccctccg 3960

aagaggtcgt gaagaagatg aagaactact ggcggcagct gctgaacgcc aagctgatta 4020

cccagagaaa gttcgacaat ctgaccaagg ccgagagagg cggcctgagc gaactggata 4080

aggccggctt catcaagaga cagctggtgg aaacccggca gatcacaaag cacgtggcac 4140

agatcctgga ctcccggatg aacactaagt acgacgagaa tgacaagctg atccgggaag 4200

tgaaagtgat caccctgaag tccaagctgg tgtccgattt ccggaaggat ttccagtttt 4260

acaaagtgcg cgagatcaac aactaccacc acgcccacga cgcctacctg aacgccgtcg 4320

tgggaaccgc cctgatcaaa aagtacccta agctggaaag cgagttcgtg tacggcgact 4380

acaaggtgta cgacgtgcgg aagatgatcg ccaagagcga gcaggaaatc ggcaaggcta 4440

ccgccaagta cttcttctac agcaacatca tgaacttttt caagaccgag attaccctgg 4500

ccaacggcga gatccggaag cggcctctga tcgagacaaa cggcgaaacc ggggagatcg 4560

tgtgggataa gggccgggat tttgccaccg tgcggaaagt gctgagcatg ccccaagtga 4620

atatcgtgaa aaagaccgag gtgcagacag gcggcttcag caaagagtct atcctgccca 4680
agaggaacag cgataagctg atcgccagaa agaaggactg ggaccctaag aagtacggcg 4740
gcttcgacag ccccaccgtg gcctattctg tgctggtggt ggccaaagtg gaaaagggca 4800
agtccaagaa actgaagagt gtgaaagagc tgctggggat caccatcatg gaaagaagca 4860
gcttcgagaa gaatcccatc gactttctgg aagccaaggg ctacaaagaa gtgaaaaagg 4920
acctgatcat caagctgcct aagtactccc tgttcgagct ggaaaacggc cggaagagaa 4980
tgctggcctc tgccggcgaa ctgcagaagg gaaacgaact ggccctgccc tccaaatatg 5040
tgaacttcct gtacctggcc agccattacg agaaactgaa agggtctcct gaggataatg 5100
aacagaagca actgtttgtc gagcagcata aacactactt ggacgaaata atcgagcaga 5160
tatcagaatt ctctaaaaga gtgattcttg cagatgccaa cctggataag gtgctcagtg 5220
cttataataa acaccgcgat aagcccatcc gcgaacaggc tgaaaatatt atacacctct 5280
ttacactcac aaatcttgga gcccccgccg cttttaaata ctttgatacc actatcgata 5340
gaaaacggta tacctcaact aaagaggttc tcgacgcaac ccttatccac cagagcatca 5400
caggactgta cgaaactagg atagatcttt cccaattggg gggcgacggt tcagaccaac 5460
ctgcaccatt ggcacgacga cgcttgcgcc tcccagggct tgacagttgt ccttcaagct 5520
tgcctgaacc ttcctctcca gccgaaccat cccccccagc cgaccccagt cctcccgcag 5580
accccggctc acccgtctgc ccagaattct aattcctaga gctcgctgat cagcctcgac 5640
tgtgccttct agttgccagc catctgttgt ttgcccctcc cccgtgcctt ccttgaccct 5700
ggaaggtgcc actcccactg tcctttccta ataaaatgag gaaattgcat cgcattgtct 5760
gagtaggtgt cattctattc tggggggtgg ggtggggcag gacagcaagg gggaggattg 5820
ggaagagaat agcaggcatg caattccctg gggagcggcc gcaggaaccc ctagtgatgg 5880
agttggccac tccctctctg cgcgctcgct cgctcactga ggccgggcga ccaaaggtcg 5940
cccgacgccc gggctttgcc cgggcggcct cagtgagcga gcgagcgcgc agctgcctgc 6000
aggggcgcct gatgcggtat tttctcctta cgcatctgtg cggtatttca caccgcatac 6060
gtcaaagcaa ccatagtacg cgccctgtag cggcgcatta agcgcggcgg gtgtggtggt 6120
tacgcgcagc gtgaccgcta cacttgccag cgccctagcg cccgctcctt tcgctttctt 6180
cccttccttt ctcgccacgt tcgccggctt tccccgtcaa gctctaaatc gggggctccc 6240
tttagggttc cgatttagtg ctttacggca cctcgacccc aaaaaacttg atttgggtga 6300
tggttcacgt agtgggccat cgccctgata gacggttttt cgccctttga cgttggagtc 6360
cacgttcttt aatagtggac tcttgttcca aactggaaca acactcaacc ctatctcggg 6420
ctattctttt gatttataag ggattttgcc gatttcggcc tattggttaa aaaatgagct 6480
gatttaacaa aaatttaacg cgaattttaa caaaatatta acgtttacaa ttttatggtg 6540

cactctcagt acaatctgct ctgatgccgc atagttaagc cagccccgac acccgccaac 6600

acccgctgac gcgccctgac gggcttgtct gctcccggca tccgcttaca gacaagctgt 6660

gaccgtctcc gggagctgca tgtgtcagag gttttcaccg tcatcaccga aacgcgcgag 6720

acgaaagggc ctcgtgatac gcctattttt ataggttaat gtcatgataa taatggtttc 6780

ttagacgtca ggtggcactt ttcggggaaa tgtgcgcgga acccctattt gtttattttt 6840

ctaaatacat tcaaatatgt atccgctcat gagacaataa ccctgataaa tgcttcaata 6900

atattgaaaa aggaagagta tgagtattca acatttccgt gtcgccctta ttcccttttt 6960

tgcggcattt tgccttcctg tttttgctca cccagaaacg ctggtgaaag taaaagatgc 7020

tgaagatcag ttgggtgcac gagtgggtta catcgaactg gatctcaaca gcggtaagat 7080

ccttgagagt tttcgccccg aagaacgttt tccaatgatg agcactttta aagttctgct 7140

atgtggcgcg gtattatccc gtattgacgc cgggcaagag caactcggtc gccgcataca 7200

ctattctcag aatgacttgg ttgagtactc accagtcaca gaaaagcatc ttacggatgg 7260

catgacagta agagaattat gcagtgctgc cataaccatg agtgataaca ctgcggccaa 7320

cttacttctg acaacgatcg gaggaccgaa ggagctaacc gcttttttgc acaacatggg 7380

ggatcatgta actcgccttg atcgttggga accggagctg aatgaagcca taccaaacga 7440

cgagcgtgac accacgatgc ctgtagcaat ggcaacaacg ttgcgcaaac tattaactgg 7500

cgaactactt actctagctt cccggcaaca attaatagac tggatggagg cggataaagt 7560

tgcaggacca cttctgcgct cggcccttcc ggctggctgg tttattgctg ataaatctgg 7620

agccggtgag cgtggaagcc gcggtatcat tgcagcactg gggccagatg gtaagccctc 7680

ccgtatcgta gttatctaca cgacggggag tcaggcaact atggatgaac gaaatagaca 7740

gatcgctgag ataggtgcct cactgattaa gcattggtaa ctgtcagacc aagtttactc 7800

atatatactt tagattgatt taaaacttca tttttaattt aaaaggatct aggtgaagat 7860

cctttttgat aatctcatga ccaaaatccc ttaacgtgag ttttcgttcc actgagcgtc 7920

agaccccgta gaaaagatca aaggatcttc ttgagatcct ttttttctgc gcgtaatctg 7980

ctgcttgcaa acaaaaaaac caccgctacc agcggtggtt tgtttgccgg atcaagagct 8040

accaactctt tttccgaagg taactggctt cagcagagcg cagataccaa atactgtcct 8100

tctagtgtag ccgtagttag gccaccactt caagaactct gtagcaccgc ctacatacct 8160

cgctctgcta atcctgttac cagtggctgc tgccagtggc gataagtcgt gtcttaccgg 8220

gttggactca agacgatagt taccggataa ggcgcagcgg tcgggctgaa cggggggttc 8280

gtgcacacag cccagcttgg agcgaacgac ctacaccgaa ctgagatacc tacagcgtga 8340

gctatgagaa agcgccacgc ttcccgaagg gagaaaggcg gacaggtatc cggtaagcgg 8400

cagggtcgga acaggagagc gcacgaggga gcttccaggg ggaaacgcct ggtatcttta 8460

tagtcctgtc gggtttcgcc acctctgact tgagcgtcga tttttgtgat gctcgtcagg 8520

ggggcggagc ctatggaaaa acgccagcaa cgcggccttt ttacggttcc tggccttttg 8580

ctggcctttt gctcacatgt 8600

<210> 33
<211> 8600
<212> DNA
<213> Artificial Sequence

<220>
<223> pX330-pNmC VECTOR DERIVED FROM STREPTOCOCCUS PYOGENES AND MUS
      MUSCLUS

<400> 33
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccatcca cggagtccca gcagccgaca agaagtacag catcggcctg gacatcggca 1380

ccaactctgt gggctgggcc gtgatcaccg acgagtacaa ggtgcccagc aagaaattca 1440

aggtgctggg caacaccgac cggcacagca tcaagaagaa cctgatcgga gccctgctgt 1500

tcgacagcgg cgaaacagcc gaggccaccc ggctgaagag aaccgccaga agaagataca 1560

ccagacggaa gaaccggatc tgctatctgc aagagatctt cagcaacgag atggccaagg 1620

tggacgacag cttcttccac agactggaag agtccttcct ggtggaagag gataagaagc 1680

acgagcggca ccccatcttc ggcaacatcg tggacgaggt ggcctaccac gagaagtacc 1740

ccaccatcta ccacctgaga aagaaactgg tggacagcac cgacaaggcc gacctgcggc 1800

tgatctatct ggccctggcc cacatgatca agttccgggg ccacttcctg atcgagggcg 1860

acctgaaccc cgacaacagc gacgtggaca agctgttcat ccagctggtg cagacctaca 1920

accagctgtt cgaggaaaac cccatcaacg ccagcggcgt ggacgccaag gccatcctgt 1980

ctgccagact gagcaagagc agacggctgg aaaatctgat cgcccagctg cccggcgaga 2040

agaagaatgg cctgttcgga aacctgattg ccctgagcct gggcctgacc cccaacttca 2100

agagcaactt cgacctggcc gaggatgcca aactgcagct gagcaaggac acctacgacg 2160

acgacctgga caacctgctg gcccagatcg gcgaccagta cgccgacctg tttctggccg 2220

ccaagaacct gtccgacgcc atcctgctga gcgacatcct gagagtgaac accgagatca 2280

ccaaggcccc cctgagcgcc tctatgatca agagatacga cgagcaccac caggacctga 2340

ccctgctgaa agctctcgtg cggcagcagc tgcctgagaa gtacaaagag attttcttcg 2400

accagagcaa gaacggctac gccggctaca ttgacggcgg agccagccag gaagagttct 2460

acaagttcat caagcccatc ctggaaaaga tggacggcac cgaggaactg ctcgtgaagc 2520

tgaacagaga ggacctgctg cggaagcagc ggaccttcga caacggcagc atcccccacc 2580

agatccacct gggagagctg cacgccattc tgcggcggca ggaagatttt tacccattcc 2640

tgaaggacaa ccgggaaaag atcgagaaga tcctgacctt ccgcatcccc tactacgtgg 2700

gccctctggc caggggaaac agcagattcg cctggatgac cagaaagagc gaggaaacca 2760

tcaccccctg gaacttcgag gaagtggtgg acaagggcgc ttccgcccag agcttcatcg 2820

agcggatgac caacttcgat aagaacctgc ccaacgagaa ggtgctgccc aagcacagcc 2880

tgctgtacga gtacttcacc gtgtataacg agctgaccaa agtgaaatac gtgaccgagg 2940

gaatgagaaa gcccgccttc ctgagcggcg agcagaaaaa ggccatcgtg gacctgctgt 3000

tcaagaccaa ccggaaagtg accgtgaagc agctgaaaga ggactacttc aagaaaatcg 3060

agtgcttcga ctccgtggaa atctccggcg tggaagatcg gttcaacgcc tccctgggca 3120

cataccacga tctgctgaaa attatcaagg acaaggactt cctggacaat gaggaaaacg 3180

aggacattct ggaagatatc gtgctgaccc tgacactgtt tgaggacaga gagatgatcg 3240

aggaacggct gaaaacctat gcccacctgt tcgacgacaa agtgatgaag cagctgaagc 3300

ggcggagata caccggctgg ggcaggctga gccggaagct gatcaacggc atccgggaca 3360

agcagtccgg caagacaatc ctggatttcc tgaagtccga cggcttcgcc aacagaaact 3420

tcatgcagct gatccacgac gacagcctga cctttaaaga ggacatccag aaagcccagg 3480

tgtccggcca gggcgatagc ctgcacgagc acattgccaa tctggccggc agccccgcca 3540

ttaagaaggg catcctgcag acagtgaagg tggtggacga gctcgtgaaa gtgatgggcc 3600

ggcacaagcc cgagaacatc gtgatcgaaa tggccagaga gaaccagacc acccagaagg 3660

gacagaagaa cagccgcgag agaatgaagc ggatcgaaga gggcatcaaa gagctgggca 3720

gccagatcct gaaagaacac cccgtggaaa acacccagct gcagaacgag aagctgtacc 3780

tgtactacct gcagaatggg cgggatatgt acgtggacca ggaactggac atcaaccggc 3840

tgtccgacta cgatgtggac catatcgtgc ctcagagctt tctgaaggac gactccatcg 3900

acaacaaggt gctgaccaga agcgacaaga accggggcaa gagcgacaac gtgccctccg 3960

aagaggtcgt gaagaagatg aagaactact ggcggcagct gctgaacgcc aagctgatta 4020

cccagagaaa gttcgacaat ctgaccaagg ccgagagagg cggcctgagc gaactggata 4080

aggccggctt catcaagaga cagctggtgg aaacccggca gatcacaaag cacgtggcac 4140

agatcctgga ctcccggatg aacactaagt acgacgagaa tgacaagctg atccgggaag 4200

tgaaagtgat caccctgaag tccaagctgg tgtccgattt ccggaaggat ttccagtttt 4260

acaaagtgcg cgagatcaac aactaccacc acgcccacga cgcctacctg aacgccgtcg 4320

tgggaaccgc cctgatcaaa aagtacccta agctggaaag cgagttcgtg tacggcgact 4380

acaaggtgta cgacgtgcgg aagatgatcg ccaagagcga gcaggaaatc ggcaaggcta 4440

ccgccaagta cttcttctac agcaacatca tgaacttttt caagaccgag attaccctgg 4500

ccaacggcga gatccggaag cggcctctga tcgagacaaa cggcgaaacc ggggagatcg 4560

tgtgggataa gggccgggat tttgccaccg tgcggaaagt gctgagcatg ccccaagtga 4620

atatcgtgaa aaagaccgag gtgcagacag gcggcttcag caaagagtct atcctgccca 4680

agaggaacag cgataagctg atcgccagaa agaaggactg ggaccctaag aagtacggcg 4740

gcttcgacag ccccaccgtg gcctattctg tgctggtggt ggccaaagtg gaaaagggca 4800

agtccaagaa actgaagagt gtgaaagagc tgctggggat caccatcatg gaaagaagca 4860

gcttcgagaa gaatcccatc gactttctgg aagccaaggg ctacaaagaa gtgaaaaagg 4920

acctgatcat caagctgcct aagtactccc tgttcgagct ggaaaacggc cggaagagaa 4980

tgctggcctc tgccggcgaa ctgcagaagg gaaacgaact ggccctgccc tccaaatatg 5040

tgaacttcct gtacctggcc agccactatg agaagctgaa gggctccccc gaggataatg 5100

agcagaaaca gctgtttgtg gaacagcaca agcactacct ggacgagatc atcgagcaga 5160

tcagcgagtt ctccaagaga gtgatcctgg ccgacgctaa tctggacaaa gtgctgtccg 5220

cctacaacaa gcaccgggat aagcccatca gagagcaggc cgagaatatc atccacctgt 5280

ttaccctgac caatctggga gcccctgccg ccttcaagta ctttgacacc accatcgacc 5340

ggaagaggta caccagcacc aaagaggtgc tggacgccac cctgatccac cagagcatca 5400

ccggcctgta cgagacacgg atcgacctgt ctcagctggg aggcgacccg agccctggtg 5460

gcctcgtggc tcctgcgttc acccggagca gcagccgcaa gcgcgcccgg cccccagccg 5520

aacccgggag tgaccagccc gcgccgctcg cgcgccggag gttacggctg cctggattgg 5580

actcctgccc cagttctctg cctgaattct aattcctaga gctcgctgat cagcctcgac 5640

tgtgccttct agttgccagc catctgttgt ttgcccctcc cccgtgcctt ccttgaccct 5700

ggaaggtgcc actcccactg tcctttccta ataaaatgag gaaattgcat cgcattgtct 5760

gagtaggtgt cattctattc tggggggtgg ggtggggcag gacagcaagg gggaggattg 5820

ggaagagaat agcaggcatg caattccctg gggagcggcc gcaggaaccc ctagtgatgg 5880

agttggccac tccctctctg cgcgctcgct cgctcactga ggccgggcga ccaaaggtcg 5940

cccgacgccc gggctttgcc cgggcggcct cagtgagcga gcgagcgcgc agctgcctgc 6000

aggggcgcct gatgcggtat tttctcctta cgcatctgtg cggtatttca caccgcatac 6060

gtcaaagcaa ccatagtacg cgccctgtag cggcgcatta agcgcggcgg gtgtggtggt 6120

tacgcgcagc gtgaccgcta cacttgccag cgccctagcg cccgctcctt tcgctttctt 6180

cccttccttt ctcgccacgt tcgccggctt tccccgtcaa gctctaaatc gggggctccc 6240

tttagggttc cgatttagtg ctttacggca cctcgacccc aaaaaacttg atttgggtga 6300

tggttcacgt agtgggccat cgccctgata gacggttttt cgccctttga cgttggagtc 6360

cacgttcttt aatagtggac tcttgttcca aactggaaca acactcaacc ctatctcggg 6420

ctattctttt gatttataag ggattttgcc gatttcggcc tattggttaa aaaatgagct 6480

gatttaacaa aaatttaacg cgaattttaa caaaatatta acgtttacaa ttttatggtg 6540

cactctcagt acaatctgct ctgatgccgc atagttaagc cagccccgac acccgccaac 6600

acccgctgac gcgccctgac gggcttgtct gctcccggca tccgcttaca gacaagctgt 6660

gaccgtctcc gggagctgca tgtgtcagag gttttcaccg tcatcaccga aacgcgcgag 6720

acgaaagggc ctcgtgatac gcctattttt ataggttaat gtcatgataa taatggtttc 6780

ttagacgtca ggtggcactt ttcggggaaa tgtgcgcgga acccctattt gtttattttt 6840

ctaaatacat tcaaatatgt atccgctcat gagacaataa ccctgataaa tgcttcaata 6900

atattgaaaa aggaagagta tgagtattca acatttccgt gtcgccctta ttcccttttt 6960

tgcggcattt tgccttcctg tttttgctca cccagaaacg ctggtgaaag taaaagatgc 7020

tgaagatcag ttgggtgcac gagtgggtta catcgaactg gatctcaaca gcggtaagat 7080

ccttgagagt tttcgccccg aagaacgttt tccaatgatg agcactttta aagttctgct 7140

atgtggcgcg gtattatccc gtattgacgc cgggcaagag caactcggtc gccgcataca 7200

ctattctcag aatgacttgg ttgagtactc accagtcaca gaaaagcatc ttacggatgg 7260

catgacagta agagaattat gcagtgctgc cataaccatg agtgataaca ctgcggccaa 7320

cttacttctg acaacgatcg gaggaccgaa ggagctaacc gcttttttgc acaacatggg 7380

ggatcatgta actcgccttg atcgttggga accggagctg aatgaagcca taccaaacga 7440

cgagcgtgac accacgatgc ctgtagcaat ggcaacaacg ttgcgcaaac tattaactgg 7500

cgaactactt actctagctt cccggcaaca attaatagac tggatggagg cggataaagt 7560

tgcaggacca cttctgcgct cggcccttcc ggctggctgg tttattgctg ataaatctgg 7620

agccggtgag cgtggaagcc gcggtatcat tgcagcactg gggccagatg gtaagccctc 7680

ccgtatcgta gttatctaca cgacggggag tcaggcaact atggatgaac gaaatagaca 7740

gatcgctgag ataggtgcct cactgattaa gcattggtaa ctgtcagacc aagtttactc 7800

atatatactt tagattgatt taaaacttca tttttaattt aaaaggatct aggtgaagat 7860

cctttttgat aatctcatga ccaaaatccc ttaacgtgag ttttcgttcc actgagcgtc 7920

agaccccgta gaaaagatca aaggatcttc ttgagatcct ttttttctgc gcgtaatctg 7980

ctgcttgcaa acaaaaaaac caccgctacc agcggtggtt tgtttgccgg atcaagagct 8040

accaactctt tttccgaagg taactggctt cagcagagcg cagataccaa atactgtcct 8100

tctagtgtag ccgtagttag gccaccactt caagaactct gtagcaccgc ctacatacct 8160

cgctctgcta atcctgttac cagtggctgc tgccagtggc gataagtcgt gtcttaccgg 8220

gttggactca agacgatagt taccggataa ggcgcagcgg tcgggctgaa cggggggttc 8280

gtgcacacag cccagcttgg agcgaacgac ctacaccgaa ctgagatacc tacagcgtga 8340

gctatgagaa agcgccacgc ttcccgaagg gagaaaggcg gacaggtatc cggtaagcgg 8400

cagggtcgga acaggagagc gcacgaggga gcttccaggg ggaaacgcct ggtatcttta 8460

tagtcctgtc gggtttcgcc acctctgact tgagcgtcga tttttgtgat gctcgtcagg 8520

ggggcggagc ctatggaaaa acgccagcaa cgcggccttt ttacggttcc tggccttttg 8580

ctggcctttt gctcacatgt 8600

<210> 34
<211> 8522
<212> DNA
<213> Artificial Sequence

<220>
<223> pX330-pNNmC VECTOR DERIVED FROM STREPTOCOCCUS PYOGENES AND MUS

MUSCLUS

<400> 34
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccatcca cggagtccca gcagccgaca agaagtacag catcggcctg gacatcggca 1380

ccaactctgt gggctgggcc gtgatcaccg acgagtacaa ggtgcccagc aagaaattca 1440

aggtgctggg caacaccgac cggcacagca tcaagaagaa cctgatcgga gccctgctgt 1500

tcgacagcgg cgaaacagcc gaggccaccc ggctgaagag aaccgccaga agaagataca 1560

ccagacggaa gaaccggatc tgctatctgc aagagatctt cagcaacgag atggccaagg 1620

tggacgacag cttcttccac agactggaag agtccttcct ggtggaagag gataagaagc 1680

acgagcggca ccccatcttc ggcaacatcg tggacgaggt ggcctaccac gagaagtacc 1740

ccaccatcta ccacctgaga aagaaactgg tggacagcac cgacaaggcc gacctgcggc 1800

tgatctatct ggccctggcc cacatgatca agttccgggg ccacttcctg atcgagggcg 1860

acctgaaccc cgacaacagc gacgtggaca agctgttcat ccagctggtg cagacctaca 1920

accagctgtt cgaggaaaac cccatcaacg ccagcggcgt ggacgccaag gccatcctgt 1980

ctgccagact gagcaagagc agacggctgg aaaatctgat cgcccagctg cccggcgaga 2040

agaagaatgg cctgttcgga aacctgattg ccctgagcct gggcctgacc cccaacttca 2100

agagcaactt cgacctggcc gaggatgcca aactgcagct gagcaaggac acctacgacg 2160

acgacctgga caacctgctg gcccagatcg gcgaccagta cgccgacctg tttctggccg 2220

ccaagaacct gtccgacgcc atcctgctga gcgacatcct gagagtgaac accgagatca 2280

ccaaggcccc cctgagcgcc tctatgatca agagatacga cgagcaccac caggacctga 2340

ccctgctgaa agctctcgtg cggcagcagc tgcctgagaa gtacaaagag attttcttcg 2400

accagagcaa gaacggctac gccggctaca ttgacggcgg agccagccag gaagagttct 2460

acaagttcat caagcccatc ctggaaaaga tggacggcac cgaggaactg ctcgtgaagc 2520

tgaacagaga ggacctgctg cggaagcagc ggaccttcga caacggcagc atcccccacc 2580

agatccacct gggagagctg cacgccattc tgcggcggca ggaagatttt tacccattcc 2640

tgaaggacaa ccgggaaaag atcgagaaga tcctgacctt ccgcatcccc tactacgtgg 2700

gccctctggc caggggaaac agcagattcg cctggatgac cagaaagagc gaggaaacca 2760

tcaccccctg gaacttcgag gaagtggtgg acaagggcgc ttccgcccag agcttcatcg 2820

agcggatgac caacttcgat aagaacctgc ccaacgagaa ggtgctgccc aagcacagcc 2880

tgctgtacga gtacttcacc gtgtataacg agctgaccaa agtgaaatac gtgaccgagg 2940

gaatgagaaa gcccgccttc ctgagcggcg agcagaaaaa ggccatcgtg gacctgctgt 3000

tcaagaccaa ccggaaagtg accgtgaagc agctgaaaga ggactacttc aagaaaatcg 3060

agtgcttcga ctccgtggaa atctccggcg tggaagatcg gttcaacgcc tccctgggca 3120

cataccacga tctgctgaaa attatcaagg acaaggactt cctggacaat gaggaaaacg 3180

aggacattct ggaagatatc gtgctgaccc tgacactgtt tgaggacaga gagatgatcg 3240

aggaacggct gaaaacctat gcccacctgt tcgacgacaa agtgatgaag cagctgaagc 3300

ggcggagata caccggctgg ggcaggctga gccggaagct gatcaacggc atccgggaca 3360

agcagtccgg caagacaatc ctggatttcc tgaagtccga cggcttcgcc aacagaaact 3420

tcatgcagct gatccacgac gacagcctga cctttaaaga ggacatccag aaagcccagg 3480

tgtccggcca gggcgatagc ctgcacgagc acattgccaa tctggccggc agccccgcca 3540

ttaagaaggg catcctgcag acagtgaagg tggtggacga gctcgtgaaa gtgatgggcc 3600

ggcacaagcc cgagaacatc gtgatcgaaa tggccagaga gaaccagacc acccagaagg 3660

gacagaagaa cagccgcgag agaatgaagc ggatcgaaga gggcatcaaa gagctgggca 3720

gccagatcct gaaagaacac cccgtggaaa acacccagct gcagaacgag aagctgtacc 3780

tgtactacct gcagaatggg cgggatatgt acgtggacca ggaactggac atcaaccggc 3840

tgtccgacta cgatgtggac catatcgtgc ctcagagctt tctgaaggac gactccatcg 3900

acaacaaggt gctgaccaga agcgacaaga accggggcaa gagcgacaac gtgccctccg 3960

aagaggtcgt gaagaagatg aagaactact ggcggcagct gctgaacgcc aagctgatta 4020

cccagagaaa gttcgacaat ctgaccaagg ccgagagagg cggcctgagc gaactggata 4080

aggccggctt catcaagaga cagctggtgg aaacccggca gatcacaaag cacgtggcac 4140

agatcctgga ctcccggatg aacactaagt acgacgagaa tgacaagctg atccgggaag 4200

tgaaagtgat caccctgaag tccaagctgg tgtccgattt ccggaaggat ttccagtttt 4260

acaaagtgcg cgagatcaac aactaccacc acgcccacga cgcctacctg aacgccgtcg 4320

tgggaaccgc cctgatcaaa aagtacccta agctggaaag cgagttcgtg tacggcgact 4380

acaaggtgta cgacgtgcgg aagatgatcg ccaagagcga gcaggaaatc ggcaaggcta 4440

ccgccaagta cttcttctac agcaacatca tgaacttttt caagaccgag attaccctgg 4500

ccaacggcga gatccggaag cggcctctga tcgagacaaa cggcgaaacc ggggagatcg 4560

tgtgggataa gggccgggat tttgccaccg tgcggaaagt gctgagcatg ccccaagtga 4620

atatcgtgaa aaagaccgag gtgcagacag gcggcttcag caaagagtct atcctgccca 4680

agaggaacag cgataagctg atcgccagaa agaaggactg ggaccctaag aagtacggcg 4740

gcttcgacag ccccaccgtg gcctattctg tgctggtggt ggccaaagtg gaaaagggca 4800

agtccaagaa actgaagagt gtgaaagagc tgctggggat caccatcatg gaaagaagca 4860

gcttcgagaa gaatcccatc gactttctgg aagccaaggg ctacaaagaa gtgaaaaagg 4920

acctgatcat caagctgcct aagtactccc tgttcgagct ggaaaacggc cggaagagaa 4980

tgctggcctc tgccggcgaa ctgcagaagg gaaacgaact ggccctgccc tccaaatatg 5040

tgaacttcct gtacctggcc agccactatg agaagctgaa gggctccccc gaggataatg 5100

agcagaaaca gctgtttgtg gaacagcaca agcactacct ggacgagatc atcgagcaga 5160

tcagcgagtt ctccaagaga gtgatcctgg ccgacgctaa tctggacaaa gtgctgtccg 5220

cctacaacaa gcaccgggat aagcccatca gagagcaggc cgagaatatc atccacctgt 5280

ttaccctgac caatctggga gcccctgccg ccttcaagta ctttgacacc accatcgacc 5340

ggaagaggta caccagcacc aaagaggtgc tggacgccac cctgatccac cagagcatca 5400

ccggcctgta cgagacacgg atcgacctgt ctcagctggg aggcgacccg agccctggtg 5460

gcctcgtggc tcctgcgttc acccggagca gcagccgcaa gcgcgcccgg cccccagccg 5520

aacccgaatt ctaattccta gagctcgctg atcagcctcg actgtgcctt ctagttgcca 5580

gccatctgtt gtttgcccct cccccgtgcc ttccttgacc ctggaaggtg ccactcccac 5640

tgtcctttcc taataaaatg aggaaattgc atcgcattgt ctgagtaggt gtcattctat 5700

tctggggggt ggggtggggc aggacagcaa gggggaggat tgggaagaga atagcaggca 5760

tgcaattccc tggggagcgg ccgcaggaac ccctagtgat ggagttggcc actccctctc 5820

tgcgcgctcg ctcgctcact gaggccgggc gaccaaaggt cgcccgacgc ccgggctttg 5880

cccgggcggc ctcagtgagc gagcgagcgc gcagctgcct gcaggggcgc ctgatgcggt 5940

attttctcct tacgcatctg tgcggtattt cacaccgcat acgtcaaagc aaccatagta 6000

cgcgccctgt agcggcgcat taagcgcggc gggtgtggtg gttacgcgca gcgtgaccgc 6060

tacacttgcc agcgccctag cgcccgctcc tttcgctttc ttcccttcct ttctcgccac 6120

gttcgccggc tttccccgtc aagctctaaa tcgggggctc cctttagggt tccgatttag 6180

tgctttacgg cacctcgacc ccaaaaaact tgatttgggt gatggttcac gtagtgggcc 6240

atcgccctga tagacggttt ttcgcccttt gacgttggag tccacgttct ttaatagtgg 6300

actcttgttc caaactggaa caacactcaa ccctatctcg ggctattctt ttgatttata 6360

agggattttg ccgatttcgg cctattggtt aaaaaatgag ctgatttaac aaaaatttaa 6420

cgcgaatttt aacaaaatat taacgtttac aattttatgg tgcactctca gtacaatctg 6480

ctctgatgcc gcatagttaa gccagccccg acacccgcca acacccgctg acgcgccctg 6540

acgggcttgt ctgctcccgg catccgctta cagacaagct gtgaccgtct ccgggagctg 6600

catgtgtcag aggttttcac cgtcatcacc gaaacgcgcg agacgaaagg gcctcgtgat 6660

acgcctattt ttataggtta atgtcatgat aataatggtt tcttagacgt caggtggcac 6720

ttttcgggga aatgtgcgcg gaacccctat ttgtttattt ttctaaatac attcaaatat 6780

gtatccgctc atgagacaat aaccctgata aatgcttcaa taatattgaa aaaggaagag 6840

tatgagtatt caacatttcc gtgtcgccct tattcccttt tttgcggcat tttgccttcc 6900

tgtttttgct cacccagaaa cgctggtgaa agtaaaagat gctgaagatc agttgggtgc 6960

acgagtgggt tacatcgaac tggatctcaa cagcggtaag atccttgaga gttttcgccc 7020

cgaagaacgt tttccaatga tgagcacttt taaagttctg ctatgtggcg cggtattatc 7080

ccgtattgac gccgggcaag agcaactcgg tcgccgcata cactattctc agaatgactt 7140

ggttgagtac tcaccagtca cagaaaagca tcttacggat ggcatgacag taagagaatt 7200

atgcagtgct gccataacca tgagtgataa cactgcggcc aacttacttc tgacaacgat 7260

cggaggaccg aaggagctaa ccgctttttt gcacaacatg ggggatcatg taactcgcct 7320

tgatcgttgg gaaccggagc tgaatgaagc cataccaaac gacgagcgtg acaccacgat 7380

gcctgtagca atggcaacaa cgttgcgcaa actattaact ggcgaactac ttactctagc 7440

ttcccggcaa caattaatag actggatgga ggcggataaa gttgcaggac cacttctgcg 7500

ctcggccctt ccggctggct ggtttattgc tgataaatct ggagccggtg agcgtggaag 7560

ccgcggtatc attgcagcac tggggccaga tggtaagccc tcccgtatcg tagttatcta 7620

cacgacgggg agtcaggcaa ctatggatga acgaaataga cagatcgctg agataggtgc 7680

ctcactgatt aagcattggt aactgtcaga ccaagtttac tcatatatac tttagattga 7740

tttaaaactt catttttaat ttaaaaggat ctaggtgaag atcctttttg ataatctcat 7800

gaccaaaatc ccttaacgtg agttttcgtt ccactgagcg tcagaccccg tagaaaagat 7860

caaaggatct tcttgagatc ctttttttct gcgcgtaatc tgctgcttgc aaacaaaaaa 7920

accaccgcta ccagcggtgg tttgtttgcc ggatcaagag ctaccaactc tttttccgaa 7980

ggtaactggc ttcagcagag cgcagatacc aaatactgtc cttctagtgt agccgtagtt 8040

aggccaccac ttcaagaact ctgtagcacc gcctacatac ctcgctctgc taatcctgtt 8100

accagtggct gctgccagtg gcgataagtc gtgtcttacc gggttggact caagacgata 8160

gttaccggat aaggcgcagc ggtcgggctg aacggggggt tcgtgcacac agcccagctt 8220

ggagcgaacg acctacaccg aactgagata cctacagcgt gagctatgag aaagcgccac 8280

gcttcccgaa gggagaaagg cggacaggta tccggtaagc ggcagggtcg gaacaggaga 8340

gcgcacgagg gagcttccag ggggaaacgc ctggtatctt tatagtcctg tcgggtttcg 8400

ccacctctga cttgagcgtc gatttttgtg atgctcgtca ggggggcgga gcctatggaa 8460

aaacgccagc aacgcggcct ttttacggtt cctggccttt tgctggcctt ttgctcacat 8520

gt 8522

<210> 35
<211> 8522
<212> DNA
<213> Artificial Sequence

<220>
<223> pX330-pNCmC VECTOR DERIVED FROM STREPTOCOCCUS PYOGENES AND MUS
 MUSCLUS

<400> 35
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccatcca cggagtccca gcagccgaca agaagtacag catcggcctg gacatcggca 1380

ccaactctgt gggctgggcc gtgatcaccg acgagtacaa ggtgcccagc aagaaattca 1440

aggtgctggg caacaccgac cggcacagca tcaagaagaa cctgatcgga gccctgctgt 1500

tcgacagcgg cgaaacagcc gaggccaccc ggctgaagag aaccgccaga agaagataca 1560

ccagacggaa gaaccggatc tgctatctgc aagagatctt cagcaacgag atggccaagg 1620

tggacgacag cttcttccac agactggaag agtccttcct ggtggaagag gataagaagc 1680

acgagcggca ccccatcttc ggcaacatcg tggacgaggt ggcctaccac gagaagtacc 1740

ccaccatcta ccacctgaga aagaaactgg tggacagcac cgacaaggcc gacctgcggc 1800

tgatctatct ggccctggcc cacatgatca agttccgggg ccacttcctg atcgagggcg 1860

acctgaaccc cgacaacagc gacgtggaca agctgttcat ccagctggtg cagacctaca 1920

accagctgtt cgaggaaaac cccatcaacg ccagcggcgt ggacgccaag gccatcctgt 1980

ctgccagact gagcaagagc agacggctgg aaaatctgat cgcccagctg cccggcgaga 2040

agaagaatgg cctgttcgga aacctgattg ccctgagcct gggcctgacc cccaacttca 2100

agagcaactt cgacctggcc gaggatgcca aactgcagct gagcaaggac acctacgacg 2160

acgacctgga caacctgctg gcccagatcg gcgaccagta cgccgacctg tttctggccg 2220

ccaagaacct gtccgacgcc atcctgctga gcgacatcct gagagtgaac accgagatca 2280

ccaaggcccc cctgagcgcc tctatgatca agagatacga cgagcaccac caggacctga 2340

ccctgctgaa agctctcgtg cggcagcagc tgcctgagaa gtacaaagag attttcttcg 2400

accagagcaa gaacggctac gccggctaca ttgacggcgg agccagccag gaagagttct 2460

acaagttcat caagcccatc ctggaaaaga tggacggcac cgaggaactg ctcgtgaagc 2520

tgaacagaga ggacctgctg cggaagcagc ggaccttcga caacggcagc atcccccacc 2580

agatccacct gggagagctg cacgccattc tgcggcggca ggaagatttt tacccattcc 2640

tgaaggacaa ccgggaaaag atcgagaaga tcctgacctt ccgcatcccc tactacgtgg 2700

gccctctggc caggggaaac agcagattcg cctggatgac cagaaagagc gaggaaacca 2760

tcaccccctg gaacttcgag gaagtggtgg acaagggcgc ttccgcccag agcttcatcg 2820

agcggatgac caacttcgat aagaacctgc ccaacgagaa ggtgctgccc aagcacagcc 2880

tgctgtacga gtacttcacc gtgtataacg agctgaccaa agtgaaatac gtgaccgagg 2940

gaatgagaaa gcccgccttc ctgagcggcg agcagaaaaa ggccatcgtg gacctgctgt 3000

tcaagaccaa ccggaaagtg accgtgaagc agctgaaaga ggactacttc aagaaaatcg 3060

agtgcttcga ctccgtggaa atctccggcg tggaagatcg gttcaacgcc tccctgggca 3120

cataccacga tctgctgaaa attatcaagg acaaggactt cctggacaat gaggaaaacg 3180

aggacattct ggaagatatc gtgctgaccc tgacactgtt tgaggacaga gagatgatcg 3240

aggaacggct gaaaacctat gcccacctgt tcgacgacaa agtgatgaag cagctgaagc 3300

ggcggagata caccggctgg ggcaggctga gccggaagct gatcaacggc atccgggaca 3360

agcagtccgg caagacaatc ctggatttcc tgaagtccga cggcttcgcc aacagaaact 3420

tcatgcagct gatccacgac gacagcctga cctttaaaga ggacatccag aaagcccagg 3480

tgtccggcca gggcgatagc ctgcacgagc acattgccaa tctggccggc agccccgcca 3540

ttaagaaggg catcctgcag acagtgaagg tggtggacga gctcgtgaaa gtgatgggcc 3600

ggcacaagcc cgagaacatc gtgatcgaaa tggccagaga gaaccagacc acccagaagg 3660

gacagaagaa cagccgcgag agaatgaagc ggatcgaaga gggcatcaaa gagctgggca 3720

gccagatcct gaaagaacac cccgtggaaa acacccagct gcagaacgag aagctgtacc 3780

tgtactacct gcagaatggg cgggatatgt acgtggacca ggaactggac atcaaccggc 3840

tgtccgacta cgatgtggac catatcgtgc ctcagagctt tctgaaggac gactccatcg 3900

acaacaaggt gctgaccaga agcgacaaga accggggcaa gagcgacaac gtgccctccg 3960

aagaggtcgt gaagaagatg aagaactact ggcggcagct gctgaacgcc aagctgatta 4020

cccagagaaa gttcgacaat ctgaccaagg ccgagagagg cggcctgagc gaactggata 4080

aggccggctt catcaagaga cagctggtgg aaacccggca gatcacaaag cacgtggcac 4140

agatcctgga ctcccggatg aacactaagt acgacgagaa tgacaagctg atccgggaag 4200

tgaaagtgat caccctgaag tccaagctgg tgtccgattt ccggaaggat ttccagtttt 4260

acaaagtgcg cgagatcaac aactaccacc acgcccacga cgcctacctg aacgccgtcg 4320

tgggaaccgc cctgatcaaa aagtacccta agctggaaag cgagttcgtg tacggcgact 4380

acaaggtgta cgacgtgcgg aagatgatcg ccaagagcga gcaggaaatc ggcaaggcta 4440

ccgccaagta cttcttctac agcaacatca tgaacttttt caagaccgag attaccctgg 4500

ccaacggcga gatccggaag cggcctctga tcgagacaaa cggcgaaacc ggggagatcg 4560

tgtgggataa gggccgggat tttgccaccg tgcggaaagt gctgagcatg ccccaagtga 4620

atatcgtgaa aaagaccgag gtgcagacag gcggcttcag caaagagtct atcctgccca 4680

agaggaacag cgataagctg atcgccagaa agaaggactg ggaccctaag aagtacggcg 4740

gcttcgacag ccccaccgtg gcctattctg tgctggtggt ggccaaagtg gaaaagggca 4800

agtccaagaa actgaagagt gtgaaagagc tgctggggat caccatcatg gaaagaagca 4860

gcttcgagaa gaatcccatc gactttctgg aagccaaggg ctacaaagaa gtgaaaaagg 4920

acctgatcat caagctgcct aagtactccc tgttcgagct ggaaaacggc cggaagagaa 4980

tgctggcctc tgccggcgaa ctgcagaagg gaaacgaact ggccctgccc tccaaatatg 5040

tgaacttcct gtacctggcc agccactatg agaagctgaa gggctccccc gaggataatg 5100

agcagaaaca gctgtttgtg gaacagcaca agcactacct ggacgagatc atcgagcaga 5160

tcagcgagtt ctccaagaga gtgatcctgg ccgacgctaa tctggacaaa gtgctgtccg 5220

cctacaacaa gcaccgggat aagcccatca gagagcaggc cgagaatatc atccacctgt 5280

ttaccctgac caatctggga gcccctgccg ccttcaagta ctttgacacc accatcgacc 5340

ggaagaggta caccagcacc aaagaggtgc tggacgccac cctgatccac cagagcatca 5400

ccggcctgta cgagacacgg atcgacctgt ctcagctggg aggcgacggg agtgaccagc 5460

ccgcgccgct cgcgcgccgg aggttacggc tgcctggatt ggactcctgc cccagttctc 5520

tgcctgaatt ctaattccta gagctcgctg atcagcctcg actgtgcctt ctagttgcca 5580

gccatctgtt gtttgcccct cccccgtgcc ttccttgacc ctggaaggtg ccactcccac 5640

tgtcctttcc taataaaatg aggaaattgc atcgcattgt ctgagtaggt gtcattctat 5700

tctggggggt ggggtggggc aggacagcaa gggggaggat tgggaagaga atagcaggca 5760

tgcaattccc tggggagcgg ccgcaggaac ccctagtgat ggagttggcc actccctctc 5820

tgcgcgctcg ctcgctcact gaggccgggc gaccaaaggt cgcccgacgc ccgggctttg 5880

cccgggcggc ctcagtgagc gagcgagcgc gcagctgcct gcaggggcgc ctgatgcggt 5940

attttctcct tacgcatctg tgcggtattt cacaccgcat acgtcaaagc aaccatagta 6000

cgcgccctgt agcggcgcat taagcgcggc gggtgtggtg gttacgcgca gcgtgaccgc 6060

tacacttgcc agcgccctag cgcccgctcc tttcgctttc ttcccttcct ttctcgccac 6120

gttcgccggc tttccccgtc aagctctaaa tcgggggctc cctttagggt tccgatttag 6180

tgctttacgg cacctcgacc ccaaaaaact tgatttgggt gatggttcac gtagtgggcc 6240

atcgccctga tagacggttt ttcgcccttt gacgttggag tccacgttct ttaatagtgg 6300

actcttgttc caaactggaa caacactcaa ccctatctcg ggctattctt ttgatttata 6360

agggattttg ccgatttcgg cctattggtt aaaaaatgag ctgatttaac aaaaatttaa 6420

cgcgaatttt aacaaaatat taacgtttac aattttatgg tgcactctca gtacaatctg 6480

ctctgatgcc gcatagttaa gccagccccg acacccgcca acacccgctg acgcgccctg 6540

acgggcttgt ctgctcccgg catccgctta cagacaagct gtgaccgtct ccgggagctg 6600

catgtgtcag aggttttcac cgtcatcacc gaaacgcgcg agacgaaagg gcctcgtgat 6660

acgcctattt ttataggtta atgtcatgat aataatggtt tcttagacgt caggtggcac 6720

ttttcgggga aatgtgcgcg gaacccctat ttgtttattt ttctaaatac attcaaatat 6780

gtatccgctc atgagacaat aaccctgata aatgcttcaa taatattgaa aaaggaagag 6840

tatgagtatt caacatttcc gtgtcgccct tattcccttt tttgcggcat tttgccttcc 6900

tgtttttgct cacccagaaa cgctggtgaa agtaaaagat gctgaagatc agttgggtgc 6960

acgagtgggt tacatcgaac tggatctcaa cagcggtaag atccttgaga gttttcgccc 7020

cgaagaacgt tttccaatga tgagcacttt taaagttctg ctatgtggcg cggtattatc 7080

ccgtattgac gccgggcaag agcaactcgg tcgccgcata cactattctc agaatgactt 7140

ggttgagtac tcaccagtca cagaaaagca tcttacggat ggcatgacag taagagaatt 7200

atgcagtgct gccataacca tgagtgataa cactgcggcc aacttacttc tgacaacgat 7260

cggaggaccg aaggagctaa ccgctttttt gcacaacatg ggggatcatg taactcgcct 7320

tgatcgttgg gaaccggagc tgaatgaagc cataccaaac gacgagcgtg acaccacgat 7380

gcctgtagca atggcaacaa cgttgcgcaa actattaact ggcgaactac ttactctagc 7440

ttcccggcaa caattaatag actggatgga ggcggataaa gttgcaggac cacttctgcg 7500

ctcggccctt ccggctggct ggtttattgc tgataaatct ggagccggtg agcgtggaag 7560

ccgcggtatc attgcagcac tggggccaga tggtaagccc tcccgtatcg tagttatcta 7620

cacgacgggg agtcaggcaa ctatggatga acgaaataga cagatcgctg agataggtgc 7680

ctcactgatt aagcattggt aactgtcaga ccaagtttac tcatatatac tttagattga 7740

tttaaaactt catttttaat ttaaaaggat ctaggtgaag atcctttttg ataatctcat 7800

gaccaaaatc ccttaacgtg agttttcgtt ccactgagcg tcagaccccg tagaaaagat 7860

caaaggatct tcttgagatc ctttttttct gcgcgtaatc tgctgcttgc aaacaaaaaa 7920

accaccgcta ccagcggtgg tttgtttgcc ggatcaagag ctaccaactc tttttccgaa 7980

ggtaactggc ttcagcagag cgcagatacc aaatactgtc cttctagtgt agccgtagtt 8040

aggccaccac ttcaagaact ctgtagcacc gcctacatac ctcgctctgc taatcctgtt 8100

accagtggct gctgccagtg gcgataagtc gtgtcttacc gggttggact caagacgata 8160

gttaccggat aaggcgcagc ggtcgggctg aacggggggt tcgtgcacac agcccagctt 8220

ggagcgaacg acctacaccg aactgagata cctacagcgt gagctatgag aaagcgccac 8280

gcttcccgaa gggagaaagg cggacaggta tccggtaagc ggcagggtcg gaacaggaga 8340

gcgcacgagg gagcttccag ggggaaacgc ctggtatctt tatagtcctg tcgggtttcg 8400

ccacctctga cttgagcgtc gatttttgtg atgctcgtca ggggggcgga gcctatggaa 8460

aaacgccagc aacgcggcct ttttacggtt cctggccttt tgctggcctt ttgctcacat 8520

gt 8522

## Description

### Technical Field

The present invention relates to a method for producing a fusion protein, a nucleic acid, a cell and an animal.

### Background Art

With a knock-out (hereafter may be referred to as "KO") mouse in which a specific gene are disabled, or a knock-in (hereafter may be referred to as "KI") mouse in which an exogenous gene are introduced into a specific locus, the function of a gene can be directly evaluated in vivo. Hence, such mice are used in many medical and life science studies.

Traditionally, KO or KI mice are created by a gene targeting method using embryonic stem (hereafter may be referred to as "ES") cells. However, a problem with this method is that production is costly and studies take years. These problems were resolved by genome editing technologies.

In genome editing, a target gene can be knocked out by introducing into a cell an artificial restriction enzyme that recognizes only a specific DNA sequence and cleaves only at that site. It is also possible to knock in an exogenous gene at a target locus by introducing an artificial restriction enzyme into a cell along with donor DNA in which the exogenous gene to be inserted is flanked by neighboring sequences at the cleavage site.

The most powerful artificial restriction enzyme that can be used for genome editing at present is the Clustered Regularly Interspaced Short Palindromic Repeats - CRISPR-Associated Protein 9 (hereinafter may be referred to as "CRISPR-Cas9") system.

Among CRISPR-Cas9s, Streptococcus pyogenes- (hereinafter may be referred to as "Sp") derived CRISPR-Cas9 (hereinafter may be referred to as "SpCas9") is widely used in studies for its ease of use and effectiveness in cleaving DNA.

The Sp-CRISPR-Cas9 system is an RNA-protein complex mainly composed of a single guide RNA (hereinafter may be referred to as "sgRNA") with the function of recognizing a target sequence and a Cas9 protein with the main function of cleaving the target sequence. A KO mouse can be obtained by simultaneously introducing the sgRNA and Cas9 protein into a fertilized mouse egg.

Further, by simultaneously cleaving upstream and downstream of a target gene region using two different sgRNAs, the region can be excised from the chromosome (excision KO). In this process, error-prone non-homologous end-joining (hereinafter may be referred to as "NHEJ") is utilized. This method has also enabled excision of millions of base pairs, which was difficult using conventional gene targeting methods.

Further, by introducing an sgRNA, a Cas9 protein and a donor DNA into a fertilized egg at the same time, a cleavage site can be repaired by homology-directed repair (hereinafter may be referred to as "HDR") using the donor DNA as a template, and a KI mouse can be obtained.

Previous studies have revealed that HDR activity is not observed in the Gi phase, increases sharply in the S phase, and decreases in the G₂/M phase. It has also been found that NHEJ activity is observed throughout the cell cycle. Therefore, studies have been conducted to control the presence of Cas9 proteins in a cell cycle-specific manner to suppress the introduction of unintended insertion or deletion mutations (Indel) by NHEJ.

For example, it is known that the human Geminin protein is degraded during the Gi phase. It is also known that the human Cdtl protein is degraded during the S/G₂ phase. In addition, Non-Patent Document 1 describes the production of a fusion protein in which a part of the human Geminin protein is linked to Cas9 (hereinafter may be referred to as "SpCas9-hGem") and a fusion protein in which a part of the human Cdtl protein is linked to Cas9 (hereinafter may be referred to as "SpCas9-hCdt1").

Non-Patent Document 1 also describes that SpCas9-hGem was degraded during the Gi phase while SpCas9-hCdt1 was degraded during the S/G₂ phase (Non-Patent Document 1, Fig. 1C, etc.).

Non-Patent Document 1 further describes that the KI efficiency at the DNMT3B locus was 17.1% by the regular SpCas9, 16.5% by SpCas9-hGem, and 9.9% by SpCas9-hCdt1 (Non-Patent Document 1, Fig. 2C, etc.).

### Citation List

### Patent Literature

Non-Patent Document 1: Howden S. E., et al., A Cas9 Variant for Efficient Generation of Indel-Free Knockin or Gene-Corrected Human Pluripotent Stem Cells, Stem Cell Reports, Vol. 7, 508-517, 2016.

### Summary of Invention

### Technical Problem

The production of KO mice and KI mice by genome editing of fertilized eggs is used in many research facilities because the cost and time are significantly reduced as compared with the method using ES cells. However, the production efficiency of excision KO mice and of HDR-dependent KI mice is not high. Therefore, an object of the present invention is to provide a modified Cas9 that improves KO efficiency and KI efficiency.

### Solution to Problem

The present invention includes the following aspects.
[1] A fusion protein including a Cas9 protein and a modifying peptide that modifies the Cas9 protein, in which the modifying peptide includes: a peptide composed of the amino acid sequence described in SEQ ID NO: 29; or a peptide composed of an amino acid sequence in which one or several amino acids are deleted, substituted, or added to the amino acid sequence described in SEQ ID NO: 29 and having activity to localize the Cas9 protein in the nucleus by forming a fusion protein with the Cas9 protein.
[2] The fusion protein according to [1], in which the modifying peptide includes: a peptide composed of the amino acid sequence described in SEQ ID NO: 1; or a peptide composed of an amino acid sequence in which one or several amino acids are deleted, substituted, or added to the amino acid sequence described in SEQ ID NO: 1 and having activity to localize the Cas9 protein in the nucleus by forming a fusion protein with the Cas9 protein.
[3] The fusion protein according to [1] or [2], in which the modifying peptide further includes: a peptide composed of the amino acid sequence described in SEQ ID NO: 2; or a peptide composed of an amino acid sequence in which one or several amino acids are deleted, substituted, or added to the amino acid sequence described in SEQ ID NO: 2 and having activity to degrade the Cas9 protein in the Gi phase by forming a fusion protein with the Cas9 protein.
[4] The fusion protein according to any one of [1] to [3], in which the modifying peptide is provided at the N-terminal, at the C-terminal, or at a site that is neither the N-terminal nor the C-terminal of the Cas9 protein.
[5] A nucleic acid encoding the fusion protein according to any one of [1] to [4].
[6] A method for producing a cell in which the genomic DNA are edited, the method including contacting the fusion protein according to any one of [1] to [4] with the genomic DNA in a cell.
[7] The method according to [6], wherein the cell is a fertilized egg.
[8] A method of producing an animal in which the genomic DNA are edited, the method including contacting the fusion protein according to any one of [1] to [4] with the genomic DNA in a fertilized egg to obtain a fertilized egg in which the genomic DNA are edited and growing the fertilized egg in which the genomic DNA are edited into an individual to obtain the animal in which the genomic DNA are edited.

### Advantageous Effects of Invention

According to an embodiment of the present invention, a modified Cas9 that improves KO efficiency and KI efficiency can be provided.

### Brief Description of Drawings

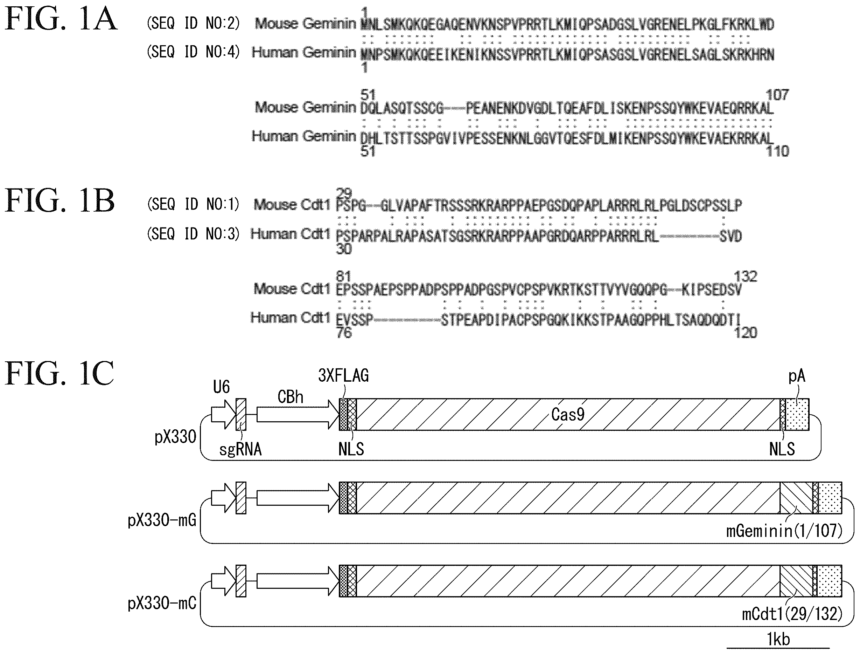

FIG. 1A is a side-by-side view of the 1st to 110th amino acid sequence of the human Geminin protein and the 1st to 107th amino acid sequence of the mouse Geminin protein. FIG. 1B is a side-by-side view of the 30th to 120th amino acid sequence of the human Cdtl protein and the 29th to 132nd amino acid sequence of the mouse Cdtl protein. In FIG. 1C, the top row is a schematic diagram illustrating the structure of a pX330 vector, the middle row is a schematic diagram illustrating the structure of a pX330-mG vector, and the bottom row is a schematic diagram illustrating the structure of a pX330-mC vector.
FIGS. 2A to 2D are fluorescence micrographs presenting the results of immunostaining in Experiment Example 2.
FIG. 3A is a schematic diagram illustrating a method for excising the Tyr gene in Experiment Example 3. FIG. 3B and FIG. 3C are photographs presenting typical mice obtained in Experiment Example 3.
FIG. 4 is a schematic diagram illustrating a method for excising the Dmd gene in Experiment Example 4.
FIG. 5A is a schematic diagram illustrating the structure of a wild-type ROSA26 locus. FIG. 5B is a schematic diagram illustrating the structure of a pRosa-CAG-fEGFP-Cables1 donor DNA plasmid used in Experiment Example 5. FIG. 5C is a schematic diagram illustrating the structure of a ROSA26 locus when a target knock-in occurs in Experiment Example 5.
FIG. 6A is a schematic diagram illustrating the structure of a wild-type Prdm14 locus. FIG. 6B is a schematic diagram illustrating the structure of a pflox-Prdm14 donor DNA plasmid used in Experiment Example 6. FIG. 6C is a schematic diagram illustrating the structure of a Prdm14 locus when a target knock-in occurs in Experiment Example 6.
FIGS. 7A to 7C are fluorescence micrographs presenting the results of immunostaining in Experiment Example 7.
FIG. 8A is a schematic diagram illustrating the structure of a pX330-mC vector. FIG. 8B is a schematic diagram illustrating the structure of a pX330-pFmC vector. FIG. 8C is a schematic diagram illustrating the structure of a pX330-pCmC vector. FIG. 8D is a schematic diagram illustrating the structure of a pX330-pMmC vector. FIG. 8E is a schematic diagram illustrating the structure of a pX330-pNmC vector. FIG. 8F is a schematic diagram illustrating the structure of a pX330-pNNmC vector. FIG. 8G is a schematic diagram illustrating the structure of a pX330-pNCmC vector.
FIG. 9 shows fluorescence micrographs presenting the results of immunostaining in Experiment Example 9.
FIG. 10A is a photograph presenting the results of detecting each one of the Cas9 fusion proteins using an anti-FLAG antibody in Experiment Example 10. FIG. 10B is a photograph presenting the results of detecting each one of the Cas9 fusion proteins using an anti-Cas9 antibody in Experiment Example 10. FIG. 10C is a photograph presenting the results of detecting the nuclear protein PARP1 using an anti-PARP1 antibody in Experiment Example 10. FIG. 10D is a photograph presenting the results of detecting the cytoplasmic protein GAPDH using an anti-GAPDH antibody in Experiment Example 10.
FIG. 11 is a diagram summarizing the structure and nuclear localization of each one of the Cas9 fusion proteins based on the results of Experiment Examples 9 and 10.
FIG. 12 shows fluorescence micrographs presenting the results of immunostaining in Experiment Example 11.

### Description of Embodiments

### Fusion Protein

In one embodiment, the present invention provides a fusion protein including a Cas9 protein and a modifying peptide that modifies the Cas9 protein, in which the modifying peptide includes: a peptide composed of the amino acid sequence described in SEQ ID NO: 1; or a peptide composed of an amino acid sequence in which one or several amino acids are deleted, substituted, or added to the amino acid sequence described in SEQ ID NO: 1 and having activity to localize the Cas9 protein in the nucleus by forming a fusion protein with the Cas9 protein.

Examples of the Cas9 protein include those derived from Streptococcus pyogenes, Staphylococcus aureus, Streptococcus thermophilus, and Geobacillus stearothermophilus. Of these, Cas9 derived from Streptococcus pyogenes (SpCas9) can be suitably used.

Examples of the modifying peptide that modifies the Cas9 protein include a peptide composed of the amino acid sequence described in SEQ ID NO: 1. A peptide composed of the amino acid sequence described in SEQ ID NO: 1 is a peptide composed of the 29th to 132nd amino acid sequence of the mouse Cdtl protein.

Cdtl is one of the licensing regulators ensuring that chromosomal replication is performed exactly once per cell cycle. Expression of Cdtl is known to be higher in the Gi phase and lower in the S phase due to ubiquitin-dependent degradation.

As described in Non-Patent Document 1, it is known that the abundance of fusion protein in which a part of the human Cdtl protein is fused to Cas9 (SpCas9-Cdt1) shows cell cycle dependence and SpCas9-Cdt1 is degraded during the S/G₂ phase.

However, contrary to expectations, as described below in the Examples, a fusion protein according to an embodiment of the present invention in which a part of the mouse Cdtl protein is fused to Cas9 (hereinafter may be referred to as "Cas9-mC") did not show cell cycle dependence and was localized in the nucleus in all phases of the cell cycle. Localization to the nucleus was confirmed not only in mouse cells but also in human cells.

In addition, as described below in the Examples, it was revealed that genome editing using Cas9-mC enables highly efficient production of a large-scale genome-deficient mouse having from several tens of kilobase pairs to several megabase pairs of genomic deficiency and of knock-in mice and flox mice which are difficult to produce.

The modifying peptide is not limited to a peptide composed of the amino acid sequence described in SEQ ID NO: 1, and may have a mutation as long as the modifying peptide has the activity to localize the Cas9 protein in the nucleus by forming a fusion protein with the Cas9 protein.

Specifically, the peptide may be composed of an amino acid sequence in which one or several amino acids are deleted, substituted, or added to the amino acid sequence described in SEQ ID NO: 1. Here, "one or several" may be, for example, from 1 to 20, from 1 to 15, from 1 to 10, from 1 to 5, or from 1 to 3.

As described below in the Examples, the present inventors found that, as a modifying peptide that modifies the Cas9 protein, a peptide composed of the amino acid sequence described in SEQ ID NO: 29 also maintains the activity to localize the Cas9 protein in the nucleus. A peptide composed of the amino acid sequence described in SEQ ID NO: 29 is a peptide composed of the 29th to 80th amino acid sequence of the mouse Cdtl protein.

A peptide composed of the amino acid sequence described in SEQ ID NO: 29 is shorter than a peptide composed of the amino acid sequence described in SEQ ID NO: 1. Therefore, the molecular weight of the fusion protein can be reduced. The size of the expression vector for the fusion protein can also be reduced. Thus, an easier-to-use fusion protein or nucleic acid encoding the fusion protein can be provided.

The modifying peptide is not limited to a peptide composed of the amino acid sequence described in SEQ ID NO: 29, and may have a mutation as long as the modifying peptide has the activity to localize the Cas9 protein in the nucleus by forming a fusion protein with the Cas9 protein.

Specifically, the peptide may be composed of an amino acid sequence in which one or several amino acids are deleted, substituted, or added to the amino acid sequence described in SEQ ID NO: 29. Here, "one or several" may be, for example, from 1 to 20, from 1 to 15, from 1 to 10, from 1 to 5, or from 1 to 3.

Hereinafter, a peptide composed of the amino acid sequence described in SEQ ID NO: 1, a peptide composed of an amino acid sequence in which one or several amino acids are deleted, substituted, or added to the amino acid sequence described in SEQ ID NO: 1, a peptide composed of the amino acid sequence described in SEQ ID NO: 29, or a peptide composed of an amino acid sequence in which one or several amino acids are deleted, substituted, or added to the amino acid sequence described in SEQ ID NO: 29 may be referred to as a "mouse Cdtl-derived peptide".

In the fusion protein according to an embodiment of the present invention, the modifying peptide may further include a peptide composed of the amino acid sequence described in SEQ ID NO: 2. The amino acid sequence described in SEQ ID NO: 2 is a peptide composed of the 1st to 107th amino acid sequence of the mouse Geminin protein.

Geminin is a licensing inhibitor that inhibits the binding of a licensing factor to the replication initiation site of a genome whose replication has started once in the S phase. Expression of Geminin is known to be high in the S/G₂/M phases but decreases once the Gi phase starts due to ubiquitin-dependent degradation.

As described below in the Examples, a fusion protein in which a part of the mouse Geminin protein is fused to Cas9 (hereinafter may be referred to as "Cas9-mG") showa cell cycle dependence, is present in the cytoplasm from the S phase to the early G₂ phase, and is degraded from the late Gi phase to the early S phase.

Thus, when the modifying peptide further includes a peptide composed of the amino acid sequence described in SEQ ID NO: 2, the modifying peptide can be degraded in the Gi phase due to ubiquitin-dependent degradation. As a result, NHEJ activity in the Gi phase can be suppressed, and the introduction of an unintended Indel mutation during a knock-in using genome editing can be suppressed.

The peptide further included by the modifying peptide is not limited to a peptide composed of the amino acid sequence described in SEQ ID NO: 2, and may have a mutation as long as the peptide has the activity to degrade the Cas9 protein in the Gi phase by forming a fusion protein with the Cas9 protein.

Specifically, the peptide may be composed of an amino acid sequence in which one or several amino acids are deleted, substituted, or added to the amino acid sequence described in SEQ ID NO: 2. Here, "one or several" may be, for example, from 1 to 20, from 1 to 15, from 1 to 10, from 1 to 5, or from 1 to 3.

Hereinafter, a peptide composed of the amino acid sequence described in SEQ ID NO: 2, or a peptide composed of an amino acid sequence in which one or several amino acids are deleted, substituted, or added in the amino acid sequence described in SEQ ID NO: 2 may be referred to as a "Geminin-derived peptide". The Geminin-derived peptide may have a part of the amino acid sequence of the human Geminin protein.

In the fusion protein of an embodiment according to the present invention, the position of the modifying peptide is not limited. For example, the position of the modifying peptide may be at the N-terminal or at the C-terminal, or at a site that is neither at the N-terminal nor the C-terminal of the Cas9 protein.

Further, in the fusion protein according to an embodiment of the present invention, the mouse Cdtl-derived peptide and the Geminin-derived peptide may be adjacent to each other or separated from each other. In addition, either the mouse Cdtl-derived peptide or the Geminin-derived peptide may be provided on the N-terminal side. That is, the Geminin-derived peptide may be provided on the C-terminal side of the mouse Cdtl-derived peptide, or the mouse Cdtl-derived peptide may be provided on the C-terminal side of the Geminin-derived peptide.

Also, when the positions of the mouse Cdtl-derived peptide and the Geminin-derived peptide are separated from each other, the mouse Cdtl-derived peptide and the Geminin-derived peptide may be provided at the N-terminal or at the C-terminal, or at a site that is neither at the N-terminal nor the C-terminal of the Cas9 protein, in a manner that is independent from each other.

For example, the Geminin-derived peptide may be provided at the N-terminal of the Cas9 protein while the mouse Cdtl-derived peptide may be provided at the C-terminal of the Cas9 protein. Alternatively, the mouse Cdtl-derived peptide may be provided at the N-terminal of the Cas9 protein while the Geminin-derived peptide may be provided at the C-terminal of the Cas9 protein.

Alternatively, either the mouse Cdtl-derived peptide or the Geminin-derived peptide may be provided at the N-terminal or the C-terminal of the Cas9 protein while the other is provided at the central portion of the Cas9 protein.

In the present specification, the N-terminal may include the vicinity of the N-terminal. Also, the C-terminal may include the vicinity of the C-terminal. That is, in some cases, even if the modifying peptide is not adjacent to the first amino acid from the N-terminal side of the Cas9 protein, when the modifying peptide is in the vicinity of the N-terminal of the Cas9 protein, the position of the modifying peptide is at the N-terminal of the Cas9 protein.

Similarly, in some cases, even if the modifying peptide is not adjacent to the first amino acid from the C-terminal side of the Cas9 protein, when the modifying peptide is in the vicinity of the C-terminal of the Cas9 protein, the position of the modifying peptide is at the C-terminal of the Cas9 protein.

Here, the vicinity may be, for example, a distance from 1 to 100 amino acids, a distance from 1 to 50 amino acids, a distance from 1 to 30 amino acids, or a distance from 1 to 20 amino acids.

The position of the modifying peptide may be a site that is neither at the N-terminal nor the C-terminal of the Cas9 protein. For example, the modifying peptide may be at the central portion of the Cas9 protein.

The fusion protein according to an embodiment of the present invention may include an additional peptide other than the Cas9 protein and the modifying peptide as long as the effect of the invention can be achieved. Examples of the additional peptide include: a tag peptide such as the FLAG tag, the His×6 tag, the MYC tag, the HA tag, and the V5 tag; a fluorescent protein such as the green fluorescent protein (GFP) and GFP derivatives; a peptide introduced in the process of genetic recombination, such as a peptide derived from the multicloning site of a vector and a peptide derived from part of a primer; and a nuclear localization signal (NLS) originally incorporated in a vector.

In the present specification, the terms "protein" and "peptide" are used without strict distinction and may be referring to a peptide or a protein depending on the number of amino acids.

### Nucleic Acid

One embodiment according to the present invention provides a nucleic acid encoding the fusion proteins described above. Introduction of the nucleic acid according to the embodiment into a cell can express the fusion protein described above. In addition, an mRNA encoding the fusion protein described above can be obtained by transcribing the nucleic acid according to the embodiment in an in vitro transcription system.

The nucleic acid according to the embodiment may be an expression vector. The expression vector is not limited, and examples include a transposon vector, a viral vector, an episomal vector, and a plasmid vector.

### Method for Producing Cell in which Genomic DNA are Edited

One embodiment according to the present invention provides a method for producing a cell in which the genomic DNA are edited, the method including contacting the fusion protein described above with genomic DNA in a cell.

The fusion protein described above is contacted with the genomic DNA along with the gRNA corresponding to a target sequence. More specifically, the fusion proteins described above can be contacted with the genomic DNA in a cell by being introduced into the cell along with the gRNA. This can result in the formation of a DNA double-strand break (DSB) in the target sequence portion.

Examples of the cell include an ES cell, an iPS cell, a fertilized egg, and a cultured cell. Examples of the cultured cell include an animal cell, an insect cell, a plant cell, and a yeast cell.

When the cell is a fertilized egg, a fertilized egg in which the genomic DNA are edited can be obtained. By growing the fertilized egg into an individual, an animal in which the genomic DNA are edited can be obtained. The animal is not limited, and examples include: a mammal such as a mouse, a rat, a sheep, a pig, a monkey, and a human; a bird such as a chicken; an amphibian such as a Xenopus laevis, a reptile such as a gecko, a fish such as a zebrafish and an Oryzias latipes, and an insect such as a Bombyx mori.

The introduction of the fusion protein and the gRNA into a cell can be accomplished by lipofection, microinjection, electroporation, and the like.

The gRNA may be a complex of a CRISPR RNA (crRNA) and a trans-activating CRISPR RNA (tracrRNA), or may be a single gRNA (sgRNA) that is a combination of a tracrRNA and a crRNA.

The gRNA can be introduced into the cell in the form of an RNA or in the form of an expression vector. Examples of a method for preparing the gRNA in the form of an RNA include: a method for synthesizing a gRNA by an in vitro transcription reaction using a construct in which a promoter such as T7 is added upstream of a nucleic acid fragment encoding the gRNA, and a method for chemically synthesizing a gRNA. When chemically synthesizing the gRNA, a chemically modified RNA may be used.

When preparing the gRNA in the form of an expression vector, the expression vector may be a plasmid vector or a viral vector that transcribes the gRNA from a Pol III promoter such as the H1 promoter or the U6 promoter. When the gRNA is expressed using an expression vector, the gRNA may be expressed constitutively or under the control of an expression-inducing promoter.

The fusion protein may be introduced into a donor cell in the form of an expression vector expressed from a Pol II promoter or in the form of a purified protein. Examples of the expression vector as the fusion protein include a transposon vector, a viral vector, an episomal vector, and a plasmid vector.

The Indel mutation may be introduced in the process in which a formed DSB is repaired by NHEJ. Alternatively, by simultaneously cleaving upstream and downstream of a target gene region using two different sgRNAs, the region can be excised from the chromosome.

A donor DNA may also be introduced into the cell along with the fusion protein and the gRNA. The donor DNA preferably includes a 5' homologous arm region, a target nucleic acid sequence region, and a 3' homologous arm region. In this case, the DSB can be repaired by homology-directed repair (HDR) using the donor DNA as a template to knock in the target nucleic acid sequence region in the donor DNA. Alternatively, the donor DNA can be knocked in by an HDR-independent method such as the PITCh method or the HITI method.

### Method for Producing Animal in which Genomic DNA are Edited

One embodiment according to the present invention provides a method of producing an animal in which the genomic DNA are edited, the method including contacting the fusion protein described above with the genomic DNA in a fertilized egg to obtain a fertilized egg in which the genomic DNA are edited and growing the fertilized egg in which the genomic DNA are edited into an individual to obtain the animal in which the genomic DNA are edited.

The steps of obtaining a fertilized egg in which the genomic DNA are edited are the same as those in the above-mentioned method for producing a cell in which the genomic DNA are edited when the cell is a fertilized egg. By growing the fertilized egg into an individual, an animal in which the genomic DNA are edited can be obtained. Examples of the animal include those described above.

The method for growing the fertilized egg in which the genomic DNA are edited into an individual can be selected depending on the species of the animal. For example, in the case of a mammal, the fertilized egg in which the genomic DNA are edited can be grown into an individual by being transplanted into the oviduct or uterus of a surrogate mother. In the case of a bird, an amphibian, a reptile, a fish, an insect, and the like, the fertilized egg in which the genomic DNA are edited can be grown into an individual by being cultured and developed in an appropriate environment.

### Examples

Hereinafter, the present invention will be described in further detail with reference to examples, but the present invention is not limited to the following examples.

### Materials and Methods

### Mouse

C57BL/J mice were purchased from Charles River Laboratories Japan, Inc. ICR mice (available from Charles River Laboratories Japan, Inc.) were used as pseudopregnant mice for producing mice. Mice were housed at a room temperature of 23.5°C ± 2.5°C and a humidity of 52.5° ± 12.5% under a light-dark cycle of 14:10 hours.

### Production of Mice

Female C57BL/6J mice at from 12 to 20 weeks of age were subcutaneously administered with 5 units of pregnant mare serum gonadotropin, and after 48 hours, intraperitoneally administered with 5 units of human chorionic gonadotropin and mated with male C57BL/6J mice. Mating was confirmed by the presence of vaginal plugs, and fertilized eggs at the pronuclear stage were collected from the oviducts. No frozen fertilized eggs or in vitro fertilized eggs were used. pX330 vector, pX330-mG vector, and pX330-mC vector were diluted to 5 ng/µl with sterilized distilled water and filtered through a PVDF membrane having a pore size of 0.22 µm before use. Various donor vectors were diluted to 10 ng/µl with sterilized distilled water and filtered through a PVDF membrane having a pore size of 0.22 µm before use. Each one of the vectors was microinjected into the male pronuclei of the pronuclear embryos. Fertilized eggs that survived from 15 minutes to 2 hours after the microinjection were transplanted into the oviducts of the pseudopregnant mice and developed into individuals.

### Analysis of Genotype

Genomic DNA was extracted from the tip of the tail of a mouse having a size of 0.5 mm or less and purified using PI-200, a genomic extraction device, and used for genotype analysis. Confirmation of the KI allele and the flox allele was performed by PCR using a PrimeSTAR GXL DNA Polymerase (available from Takara Bio Inc.). Confirmation of the KO allele was performed by PCR using an AmpliTaq Gold 360 Master Mix (available from Thermo Fisher Scientific Inc.).

### Experiment Example 1

### Production 1 of Cas9 Fusion Protein Expression Vector

pX330 (Plasmid #42230, available from Addgene) is a vector capable of expressing both sgRNA and Cas9. The top row of FIG. 1C illustrates the structure of the pX330 vector. In the pX330 vector, sgRNA is expressed by the U6 promoter. Furthermore, the SpCas9 protein is expressed by the CBh promoter. The N-terminal and the C-terminal of the SpCas9 protein are each linked to a nuclear localization signal (NLS) derived from the SV40 virus. In addition, the N-terminal side of the NLS at the N-terminal is linked to a 3xFLAG tag peptide.

A Cas9 fusion protein expression vector was prepared based on the pX330 vector. More specifically, a vector (SEQ ID NO: 5, hereinafter may be referred to as "pX330-mG") expressing a fusion protein (hereinafter may be referred to as "Cas9-mG") in which the C-terminal of the SpCas9 protein was linked to a peptide composed of the 1st to 107th of a Geminin protein derived from a mouse rather than a human was prepared.

FIG. 1A is a side-by-side view of the 1st to 110th amino acid sequence of the human Geminin protein (SEQ ID NO: 4) and the 1st to 107th amino acid sequence of the mouse Geminin protein (SEQ ID NO: 2). Also, the structure of the pX330-mG vector is illustrated in the middle row of FIG. 1C.

In addition, a vector (SEQ ID NO: 6, hereinafter may be referred to as "pX330-mC") expressing a fusion protein (hereinafter may be referred to as "Cas9-mC") in which the C-terminal of the SpCas9 protein was linked to a peptide composed of the 29th to 132nd of a Cdtl protein derived from a mouse rather than a human was prepared.

FIG. 1B is a side-by-side view of the 30th to 120th amino acid sequence of the human Cdtl protein (SEQ ID NO: 3) and the 29th to 132nd amino acid sequence of the mouse Cdtl protein (SEQ ID NO: 1). Also, the structure of the pX330-mC vector is illustrated in the bottom row of FIG. 1C.

### Experiment Example 2

### Study of Cell Cycle Dependence and Subcellular Localization of Cas9, Cas9-mG, and Cas9-mC

Fertilized mouse eggs were used to study cell cycle dependence and subcellular localization of Cas9-mG protein and Cas9-mC protein expressed by the introduction of the expression vectors prepared in Experiment Example 1.

First, using an in vitro transcription system, Cas9-mG mRNA, Cas9-mC mRNA, and Cas9 mRNA were prepared separately by transcribing each one of the expression vectors prepared in Experiment Example 1.

Then, the Cas9-mG mRNA and the Cas9-mC mRNA were each injected into the cytoplasm of fertilized mouse eggs obtained via intracytoplasmic sperm injection. For comparison, the same study was conducted on both fertilized mouse eggs injected with regular Cas9 mRNA and fertilized mouse eggs injected with nothing.

As described above, the Cas9-mG protein, the Cas9-mC protein and the regular Cas9 protein encoded by each one of the vectors prepared in Experiment Example 1 are each linked to a 3×FLAG tag peptide. Therefore, the Cas9-mG protein, the Cas9-mC protein and the regular Cas9 protein translated from the Cas9-mG mRNA, the Cas9-mC mRNA and the Cas9 mRNA injected into the fertilized mouse eggs in this Experiment Example are also each linked to a 3×FLAG tag peptide. Thus, each one of the Cas9 proteins can be detected by immunostaining using an anti-FLAG antibody.

The fertilized eggs were then cultured in vitro, and the 2-cell stage embryos from the late Gi phase to the early S phase and the 2-cell stage embryos from the late S phase to the early G₂ phase were immunostained with the anti-FLAG antibody and observed by fluorescence microscopy.

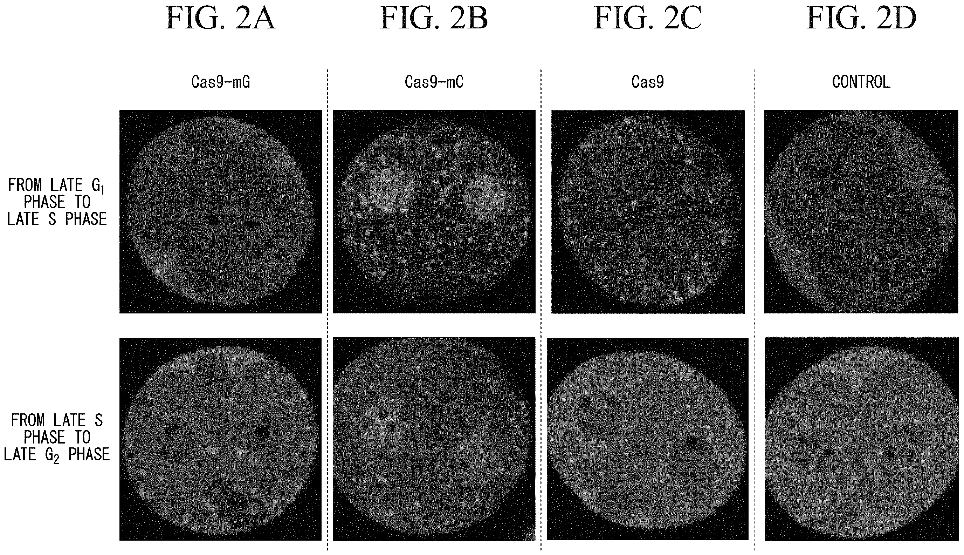

FIGS. 2A to 2D are fluorescence micrographs presenting the results of immunostaining. FIG. 2A is the result of the Cas9-mG protein, FIG. 2B is the result of the Cas9-mC protein, FIG. 2C is the result of the regular Cas9 protein, and FIG. 2D is a control in which nothing is introduced.

As a result, the regular Cas9 protein was detected as a dot-like signal in the cytoplasm at any phase of the cell cycle. As described above, the N-terminal and the C-terminal of the Cas9 protein used in this Experimental Example are each linked to a nuclear localization signal derived from the SV40 virus; however, no nuclear localization of the Cas9 protein was observed.

Meanwhile, it was confirmed that the Cas9-mG protein was not detected in the late Gi phase to the early S phase and was degraded. The Cas9-mG protein was also detected as a dot-like signal in the cytoplasm from the S phase to the early G₂ phase. As described above, the N-terminal and the C-terminal of the Cas9-mG protein used in this Experimental Example are each linked to a nuclear localization signal derived from the SV40 virus; however, no nuclear localization of the Cas9 protein was observed.

In addition, contrary to expectations, cell cycle dependence was not confirmed for the Cas9-mC protein, and signals were detected in all phases of the cell cycle. Furthermore, the nuclei were stained strongly in all phases of the cell cycle. This result revealed that the Cas9-mC protein showed high nuclear localization.

### Experiment Example 3

### Production 1 of Large-scale Genome-deficient Mouse using Cas9-mC

Investigation regarding whether Cas9-mC would increase the efficiency of excision of large-scale genomic regions was conducted.

Specifically, Cas9-mC was used to produce mice in which the Tyr gene was fully excised (excision KO mice). Also, for comparison, mice in which the Tyr gene was fully excised were prepared by the same method except that the regular Cas9 was used. The Tyr gene is a gene that dominates the fur color of a mouse, and a mouse lacking both alleles of the Tyr gene exhibits the albino trait. Note that a mouse lacking one allele of the Tyr gene does not exhibit the albino trait.

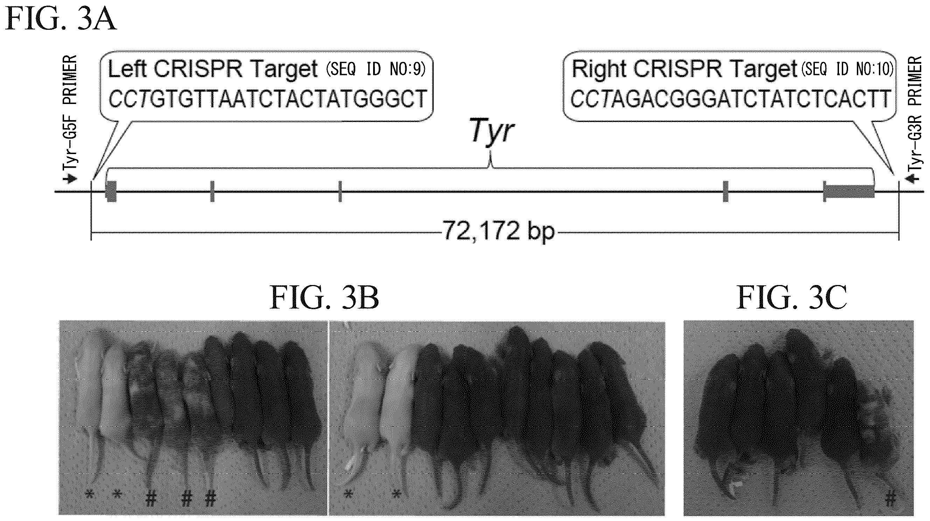

FIG. 3A is a schematic diagram illustrating a method for excising the Tyr gene. In FIG. 3A, "Tyr-G5F primer" (SEQ ID NO: 7) and "Tyr-G3R primer" (SEQ ID NO: 8) are the primers used to confirm the genotype of a mouse.

Specifically, a pX330-mC-Tyr-L vector that expresses an sgRNA targeting the genomic sequence upstream of the Tyr gene (Left CRISPR Target, SEQ ID NO: 9) and the Cas9-mC protein, as well as a pX330-mC-Tyr-R vector that expresses an sgRNA targeting the genomic sequence downstream of the Tyr gene (Right CRISPR Target, SEQ ID NO: 10) and the Cas9-mC protein were prepared.

Then, a pX330-Tyr-L vector that expresses an sgRNA targeting the genomic sequence upstream of the Tyr gene (Left CRISPR Target, SEQ ID NO: 9) and the regular Cas9 protein, as well as a pX330-Tyr-R vector that expresses an sgRNA targeting the genomic sequence downstream of the Tyr gene (Right CRISPR Target, SEQ ID NO: 10) and the regular Cas9 protein were prepared. The distance between the Left CRISPR Target (SEQ ID NO: 9) and the Right CRISPR Target (SEQ ID NO: 10) was 72,172 base pairs.

Then, the male pronuclei of fertilized eggs (at the pronuclear stage) of C57BL6/J mice with black fur obtained by natural mating were respectively microinjected with a mixture of the pX330-mC-Tyr-L vector (5 ng/µL) and the pX330-mC-Tyr-R vector (5 ng/µL) and a mixture of the pX330-Tyr-L vector (5 ng/µL) and the pX330-Tyr-R vector (5 ng/µL). Subsequently, the fertilized eggs with the vectors introduced were transplanted into the oviducts of pseudopregnant mice and developed into mice, and the fur color of the resulting mice was observed.

FIGS. 3B and 3C are photographs presenting typical mice obtained. FIG. 3B is a photograph of mice developed from fertilized eggs with Cas9-mC introduced, while FIG. 3C is a photograph of mice developed from fertilized eggs with regular Cas9 introduced. In FIGS. 3B and 3C, "*" indicates mice showing a complete albino trait, while "#" indicates mice showing a mosaic albino trait.

It is believed that a complete albino trait is the result of a full-length deletion of the Tyr gene in both alleles in perhaps the 1-cell stage of a fertilized egg, while a mosaic albino trait is the result of a full-length deletion of the Tyr gene in both alleles of either cell from the 2-cell stage onward of a fertilized egg.

As a result, among the 34 mice obtained from the fertilized eggs with Cas9-mC introduced, 5 showed the complete albino trait while 3 showed the mosaic albino trait. In addition, analysis of the genotype by PCR revealed that 17 (50.0%) of the 34 mice developed from the fertilized eggs with Cas9-mC introduced had alleles with the Tyr gene completely excised.

Further, among the 21 mice obtained from the fertilized eggs with the regular Cas9 introduced, 1 showed the mosaic albino trait while none showed the complete albino trait. In addition, analysis of the genotype by PCR revealed that 6 (28.6%) of the 21 mice developed from the fertilized eggs with the regular Cas9 introduced had alleles with the Tyr gene completely excised. Table 1 below shows the results of producing the large-scale Tyr-deficient mice.

The above results show that by using Cas9-mC, a large-scale genome-deficient mouse having several tens of kilobase pairs of genomic deficiency can be produced with high efficiency.

**Table 1**

| | Cas9-mC | Cas9 |
|---|---|---|
| Number of fertilized eggs with vectors introduced | 102 | 107 |
| Number of fertilized eggs transplanted into pseudopregnant mice | 100 | 100 |
| Total number of newborns | 34 | 21 |
| Number of newborns with large-scale deficient alleles | 17 (50.0%)^{a} | 6 (28.6%)^{a} |
| Number of newborns showing complete albino trait | 5 | 0 |
| Number of newborns showing mosaic albino trait | 3 | 1 |

| | | |
|---|---|---|
| ^{a}: Number of newborns with large-scale deficient alleles/Total number of newborns × 100 | | |

### Experiment Example 4

### Production 2 of Large-scale Genome-deficient Mouse using Cas9-mC

Investigation regarding whether Cas9-mC would increase the efficiency of excision of large-scale genomic regions was conducted.

Specifically, Cas9-mC was used to produce mice in which the Dmd gene was fully excised (excision KO mice). The Dmd gene is known to be the longest gene encoding a protein. Also, for comparison, mice in which the Dmd gene was fully excised were prepared by the same method except that regular Cas9 was used. Because the Dmd gene is located on the X chromosome, the male has 1 copy and the female has 2 copies.

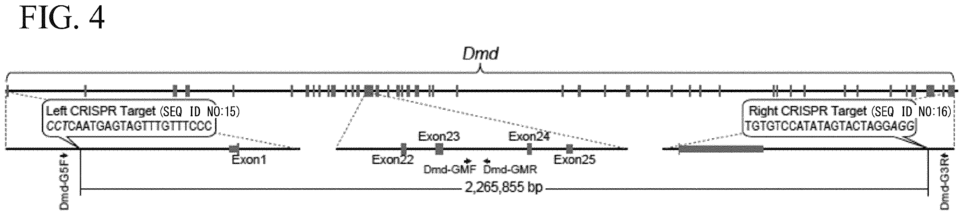

FIG. 4 is a schematic diagram illustrating a method for excising the Dmd gene. In FIG. 4, "Dmd-G5F" (SEQ ID NO: 11), "Dmd-GMF" (SEQ ID NO: 12), "Dmd-GMR" (SEQ ID NO: 13), and "Dmd-G3R" (SEQ ID NO: 14) are the primers used to confirm the genotype of a mouse.

Specifically, first, a pX330-mC-Dmd-L vector that expresses an sgRNA targeting the genomic sequence upstream of the Dmd gene (Left CRISPR Target, SEQ ID NO: 15) and the Cas9-mC protein, as well as a pX330-mC-Dmd-R vector that expresses an sgRNA targeting the genomic sequence downstream of the Dmd gene (Right CRISPR Target, SEQ ID NO: 16) and the Cas9-mC protein were prepared.

Then, a pX330-Dmd-L vector that expresses an sgRNA targeting the genomic sequence upstream of the Dmd gene (Left CRISPR Target, SEQ ID NO: 15) and the regular Cas9 protein, as well as a pX330-Dmd-R vector that expresses an sgRNA targeting the genomic sequence downstream of the Dmd gene (Right CRISPR Target, SEQ ID NO: 16) and the regular Cas9 protein were prepared. The distance between the Left CRISPR Target (SEQ ID NO: 15) and the Right CRISPR Target (SEQ ID NO: 16) was 2,265,855 base pairs.

Then, the male pronuclei of fertilized eggs (at the pronuclear stage) of C57BL6/J mice obtained by natural mating were respectively microinjected with a mixture of the pX330-mC-Dmd-L vector (5 ng/µL) and the pX330-mC-Dmd-R vector (5 ng/µL) and a mixture of the pX330-Dmd-L vector (5 ng/µL) and the pX330-Dmd-R vector (5 ng/µL). Subsequently, the fertilized eggs with the vectors introduced were transplanted into the oviducts of pseudopregnant mice and developed into mice, of which the genotypes were confirmed by PCR.

As a result, a total of 68 mice were obtained from the fertilized eggs with Cas9-mC introduced. Furthermore, analysis of the genotype by PCR revealed that 11 (16.2%) of the 68 mice developed from the fertilized eggs with Cas9-mC introduced had alleles with the Dmd gene completely excised.

In addition, a total of 70 mice were obtained from the fertilized eggs with the regular Cas9 induced. Furthermore, analysis of the genotype by PCR revealed that 6 (8.6%) of the 70 mice developed from the fertilized eggs with the regular Cas9 introduced had alleles with the Dmd gene completely excised.

It is worth noting that the 6 mice with the full Dmd excision knock-out alleles obtained by the introduction of regular Cas9 were mosaic or heterozygous variants that also retained other Dmd alleles, while 5 male mice and 1 female mouse with the full Dmd excision knock-out alleles obtained by the introduction of Cas9-mC were individuals with full deletion of the Dmd gene having only the full Dmd knock-out allele. Table 2 below shows the results of producing the large-scale Dmd-deficient mice.

The above results show that by using Cas9-mC, a large-scale genome-deficient mouse having several megabase pairs of genomic deficiency can be produced with high efficiency.

**Table 2**

| | Cas9-mC | Cas9 |
|---|---|---|
| Number of fertilized eggs with vectors introduced | 165 | 166 |
| Number of fertilized eggs transplanted into pseudopregnant mice | 149 | 154 |
| Total number of newborns | 68 | 70 |
| Number of newborns with large-scale deficient alleles | 11 (16.2%)^{a} | 6 (8.6%)^{a} |
| Number of newborns with only large-scale deficient alleles | 6 (♂:5, ♀:1) | 0 |

| | | |
|---|---|---|
| ^{a}: Number of newborns with large-scale deficient alleles/Total number of newborns × 100 | | |

### Experiment Example 5

### Production 1 of Knock-in Mouse using Cas9-mC

In genome editing of fertilized eggs, it is known that it is difficult to produce a mouse knocked in with a gene fragment including a base sequence having a high GC content and a flox mouse in which LoxP sequences must be introduced into two sites at the same time. Investigation regarding whether Cas9-mC is useful for producing such knock-in mice or flox mice which are difficult to produce was conducted.

First, an experiment was conducted in which an expression cassette CAG-flox EGFP-Cables1 including a Cables 1 gene cDNA having a CAG promoter containing a base sequence with a high GC content and a base sequence with a high GC content were knocked in. The locus of knock-in was also a ROSA26 locus in which the 5' homologous arm region includes a base sequence having a high GC content.

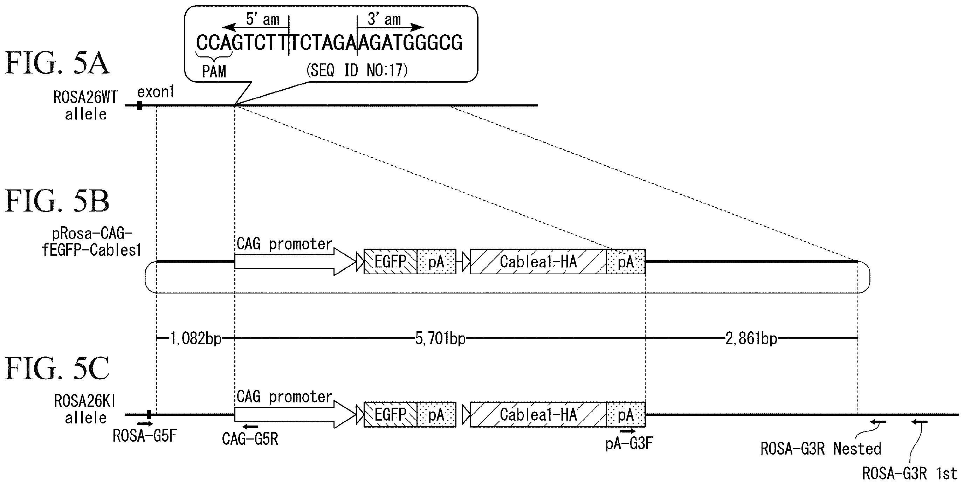

FIGS. 5A to 5C are schematic diagrams, illustrating a method for knocking a CAG-flox EGFP-Cablesl gene fragment into the ROSA26 locus. FIG. 5A is a schematic diagram illustrating the structure of a wild-type ROSA26 locus. FIG. 5A also illustrates the positions of the target sequence of sgRNA (SEQ ID NO: 17), 5' homologous arm region and 3' homologous arm region.

FIG. 5B is a schematic diagram illustrating the structure of a pRosa-CAG-fEGFP-Cables1 donor DNA plasmid. FIG. 5C is a schematic diagram illustrating the structure of the ROSA26 locus when the target knock-in occurs. In FIG. 5C, "ROSA-G5F" (SEQ ID NO: 18), "CAG-G5R" (SEQ ID NO: 19), "pA-G3F" (SEQ ID NO: 20), "ROSA-G3R Nested" (SEQ ID NO: 21), and "ROSA-G3R 1st" (SEQ ID NO: 22) are the primers used to confirm the genotype of a mouse.

Specifically, first, a pX330-mC-ROSA vector that expresses an sgRNA targeting the first intron of the ROSA26 locus and the Cas9-mC protein, as well as a pX330-ROSA vector that expresses an sgRNA targeting the first intron of the ROSA26 locus and the regular Cas9 protein were prepared.

Then, the male pronuclei of fertilized eggs (at the pronuclear stage) of C57BL6/J mice obtained by natural mating were respectively microinjected with a mixture of the pX330-mC-ROSA vector (5 ng/µL) and the pRosa-CAG-fEGFP-Cablesl vector (10 ng/µL) and a mixture of the pX330-ROSA vector (5 ng/µL) and the pRosa-CAG-fEGFP-Cablesl vector (10 ng/µL). Subsequently, the fertilized eggs with the vectors introduced were transplanted into the oviducts of pseudopregnant mice and developed into mice, of which the genotypes were confirmed by PCR.

As a result, a total of 19 mice were obtained from the fertilized eggs with Cas9-mC introduced. Furthermore, analysis of the genotype by PCR revealed that 4 (21.1%) of the 19 mice developed from the fertilized eggs with Cas9-mC introduced had the target knock-in alleles.

In addition, a total of 44 mice were obtained from the fertilized eggs into which the regular Cas9 was introduced, but analysis of the genotype by PCR revealed that no mouse having the target knock-in alleles was obtained. Table 3 below shows the results of producing the knock-in mice.

The above results indicate that by using Cas9-mC, it is possible to produce KI mice and flox mice which are difficult to produce.

**Table 3**

| | Cas9-mC | Cas9 |
|---|---|---|
| Number of fertilized eggs with vectors introduced | 165 | 122 |
| Number of fertilized eggs transplanted into pseudopregnant mice | 154 | 106 |
| Total number of newborns or embryos^{b} of which the genotype was analyzed | 19 | 44 |
| Number of newborns or embryos^{b} with the knock-in alleles | 4 (21.1%)^{a} | 0 (0.0%)^{a} |

| | | |
|---|---|---|
| ^{a}: Number of newborns or embryos with the knock-in alleles/Total number of newborns or embryos of which the genotype was analyzed × 100 ^{b}: Embryo that is 18.5 days after fertilization | | |

### Experiment Example 6

### Production 2 of Knock-in Mouse using Cas9-mC

Then, LoxP sequences were introduced upstream and downstream of the sixth exon of the Prdm14 gene to produce a Prdm14 flox mouse.

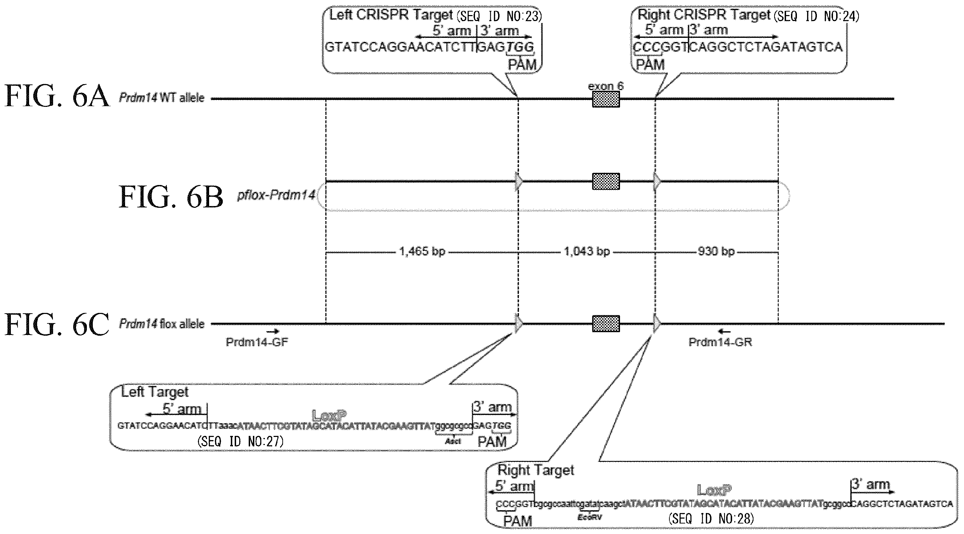

FIGS. 6A to 6C are schematic diagrams illustrating a method for knocking the LoxP sequences into the Prdm14 locus. FIG. 6A is a schematic diagram illustrating the structure of a wild-type Prdm14 locus.

FIG. 6A also illustrates the positions of the sgRNA targeting the genomic sequence upstream of the sixth exon of the Prdm14 gene (Left CRISPR Target, SEQ ID NO: 23), the sgRNA targeting the genomic sequence downstream of the sixth exon of the Prdm14 gene (Right CRISPR Target, SEQ ID NO: 24), the 5' homologous arm region and the 3' homologous arm region.

FIG. 6B is a schematic diagram illustrating the structure of a pflox-Prdm14 donor DNA plasmid. FIG. 6C is a schematic diagram illustrating the structure of the Prdm14 locus when the target knock-in occurs. In FIG. 6C, "Prdm14-GF" (SEQ ID NO: 25) and "Prdm14-GR" (SEQ ID NO: 26) are the primers used to confirm the genotype of a mouse, while "LoxP" represents the LoxP sequence knocked in.

Specifically, first, a pX330-mC-Prdm14-L vector that expresses an sgRNA targeting the genomic sequence upstream of the Prdm14 gene (Left CRISPR Target, SEQ ID NO: 23) and the Cas9-mC protein, as well as a pX330-mC-Prdm14-R vector that expresses an sgRNA targeting the genomic sequence downstream of the Prdm14 gene (Right CRISPR Target, SEQ ID NO: 24) and the Cas9-mC protein were prepared.

Then, a pX330-Prdm14-L vector that expresses an sgRNA targeting the genomic sequence upstream of the Prdm14 gene (Left CRISPR Target, SEQ ID NO: 23) and the regular Cas9 protein, as well as a pX330-Prdm14-R vector that expresses an sgRNA targeting the genomic sequence downstream of the Prdm14 gene (Right CRISPR Target, SEQ ID NO: 24) and the regular Cas9 protein were prepared.

Then, the male pronuclei of fertilized eggs (at the pronuclear stage) of C57BL6/J mice obtained by natural mating were respectively microinjected with a mixture of the pX330-mC-Prdm14-L vector (5 ng/µL), the pX330-mC-Prdm14-R vector (5 ng/µL), and the pflox-Prdm14 vector (10 ng/µL), as well as a mixture of the pX330-Prdm14-L vector (5 ng/µL), the pX330-Prdm14-R vector (5 ng/µL), and the pflox-Prdm14 vector (10 ng/µL). Subsequently, the fertilized eggs with the vectors introduced were transplanted into the oviducts of pseudopregnant mice and developed into mice, of which the genotypes were confirmed by PCR.

As a result, a total of 22 mice were obtained from the fertilized eggs with Cas9-mC introduced. Furthermore, analysis of the genotype by PCR revealed that 4 (18.2%) of the 22 mice developed from the fertilized eggs with Cas9-mC introduced had the target knock-in alleles.

In addition, a total of 25 mice were obtained from the fertilized eggs into which the regular Cas9 was introduced, but analysis of the genotype by PCR revealed that no mouse having the target knock-in alleles was obtained. Table 4 below shows the results of producing the knock-in mice.

**Table 4**

| | Cas9-mC | Cas9 |
|---|---|---|
| Number of fertilized eggs with vectors introduced | 104 | 104 |
| Number of fertilized eggs transplanted into pseudopregnant mice | 87 | 85 |
| Number of newborns grown until weaning | 22 | 25 |
| Number of newborns with the knock-in alleles | 4 (18.2%)^{a} | 0 (0.0%)^{a} |

| | | |
|---|---|---|
| ^{a}: Number of newborns with the knock-in alleles/Total number of newborns grown until weaning × 100 | | |

### Experiment Example 7

### Study of Subcellular Localization of Cas9, Cas9-mG, Cas9-mC

HEK293T cells, which are cells derived from human fetal kidney, were used to study subcellular localization of the Cas9-mG protein and the Cas9-mC protein expressed by the introduction of the expression vectors prepared in Experiment Example 1.

First, the pX330-mG vector and the pX330-mC vector were introduced into HEK293T cells, respectively. HEK293T cells with the pX330 vector introduced were also prepared for comparison.

As described above, the Cas9-mG protein, the Cas9-mC protein, and the regular Cas9 protein encoded by each one of the vectors are each linked to a 3×FLAG tag peptide. Thus, each one of the Cas9 proteins can be detected by immunostaining using an anti-FLAG antibody.

Each cell was then formaldehyde-fixed and immunostained with an anti-FLAG antibody. The nuclei were stained with 4',6-diamidino-2-phenylindole (DAPI). Each stained cell was then observed using a fluorescence microscope.

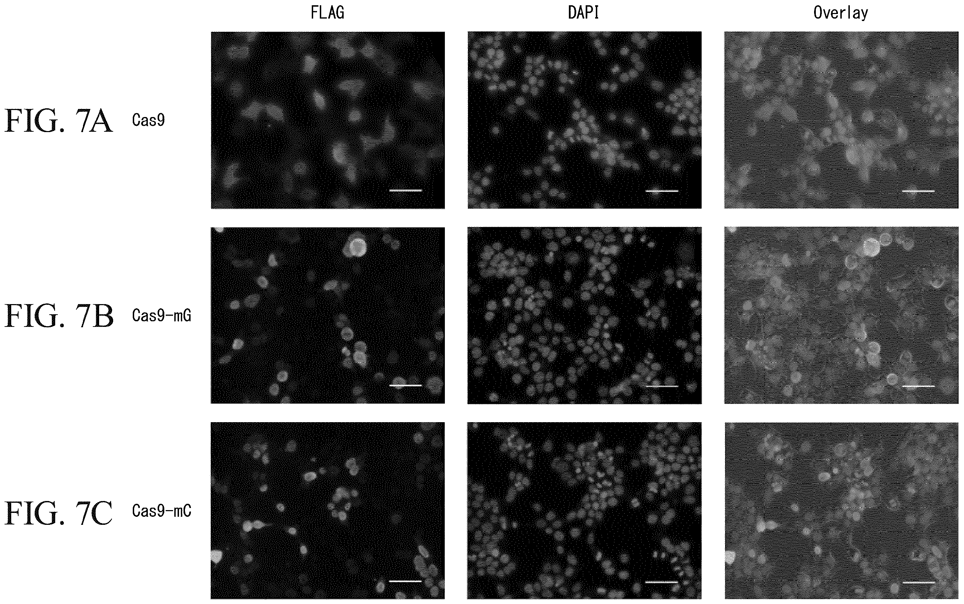

FIGS. 7A to 7C are fluorescence micrographs presenting the results of immunostaining. FIG. 7A is the result of the regular Cas9 protein, FIG. 7B is the result of the Cas9-mG protein, and FIG. 7C is the result of the Cas9-mC protein. In FIG. 7, "Overlay" is the result of synthesizing the staining result using the anti-FLAG antibody and the staining result using DAPI.

As a result, the regular Cas9 protein was detected in the cytoplasm. As described above, the N-terminal and the C-terminal of the Cas9 protein used in this Experimental Example are each linked to a nuclear localization signal derived from the SV40 virus; however, no nuclear localization of the Cas9 protein was observed.

Meanwhile, the Cas9-mG protein was found to be localized in the nucleus. The Cas9-mC protein was also found to be localized in the nucleus. These results revealed that the Cas9-mC protein showed high nuclear localization even in human cells.

### Experiment Example 8

### Production 2 of Cas9 Fusion Protein Expression Vector

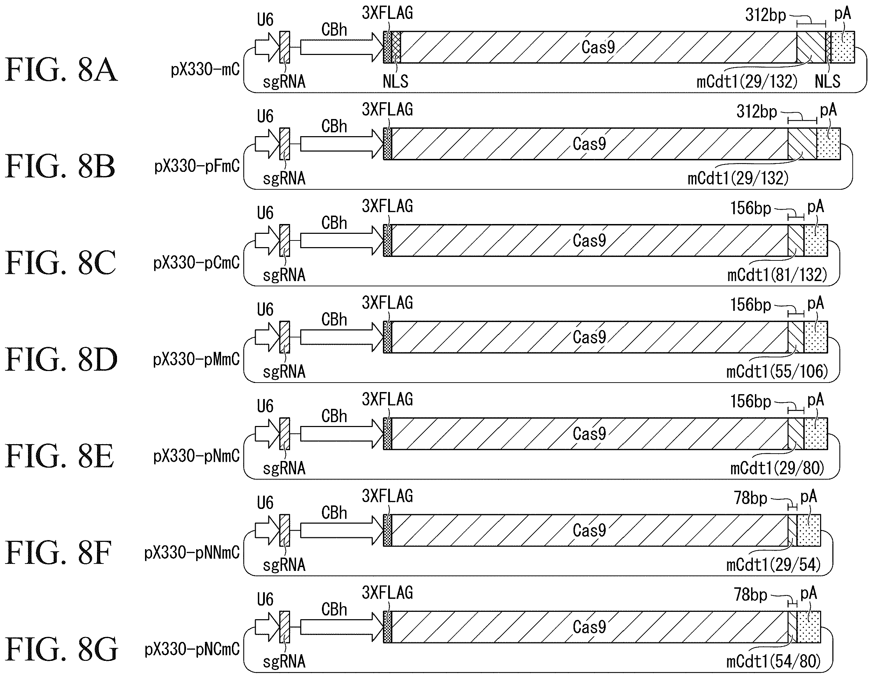

The N-terminal and the C-terminal of the SpCas9 protein expressed by the pX330-mC vector prepared in Experiment Example 1 each had a nuclear transition signal (NLS) derived from the SV40 virus. FIG. 8A illustrates the structure of the pX330-mC vector.

In this Experiment Example, a vector (SEQ ID NO: 30, hereinafter may be referred to as "pX330-pFmC") that removes these NLS present at the N-terminal and the C-terminal of the SpCas9 protein and that expresses a fusion protein (hereinafter may be referred to as "Cas9-pFmC") in which the C-terminal of the SpCas9 protein was linked to a peptide composed of the 29th to 132nd of the Cdtl protein derived from a mouse was prepared. FIG. 8B illustrates the structure of the pX330-pFmC vector.

Furthermore, in this Experiment Example, fusion proteins in which the position and length of the mouse Cdtl-derived peptide linked to the C-terminal of the SpCas9 protein were variously modified were prepared. Specifically, a vector (SEQ ID NO: 31, hereinafter may be referred to as "pX330-pCmC") expressing a fusion protein (hereinafter may be referred to as "Cas9-pCmC") having no nuclear localization signal derived from the SV40 virus and in which the C-terminal of the SpCas9 protein was linked to a peptide composed of the 81st to 132nd of the Cdtl protein derived from a mouse was prepared. FIG. 8C illustrates the structure of the pX330-pCmC vector.

In addition, a vector (SEQ ID NO: 32, hereinafter may be referred to as "pX330-pMmC") expressing a fusion protein (hereinafter may be referred to as "Cas9-pMmC") having no nuclear localization signal derived from the SV40 virus and in which the C-terminal of the SpCas9 protein was linked to a peptide composed of the 55th to 106th of the Cdtl protein derived from a mouse was prepared. FIG. 8D illustrates the structure of the pX330-pMmC vector.

In addition, a vector (SEQ ID NO: 33, hereinafter may be referred to as "pX330-pNmC") expressing a fusion protein (hereinafter may be referred to as "Cas9-pNmC") having no nuclear localization signal derived from the SV40 virus and in which the C-terminal of the SpCas9 protein was linked to a peptide composed of the 29th to 80th of the Cdtl protein derived from a mouse was prepared. FIG. 8E illustrates the structure of the pX330-pNmC vector.

In addition, a vector (SEQ ID NO: 34, hereinafter may be referred to as "pX330-pNNmC") expressing a fusion protein (hereinafter may be referred to as "Cas9-pNNmC") having no nuclear localization signal derived from the SV40 virus and in which the C-terminal of the SpCas9 protein was linked to a peptide composed of the 29th to 54th of the Cdtl protein derived from a mouse was prepared. FIG. 8F illustrates the structure of the pX330-pNNmC vector.

In addition, a vector (SEQ ID NO: 35, hereinafter may be referred to as "pX330-pNCmC") expressing a fusion protein (hereinafter may be referred to as "Cas9-pNCmC") having no nuclear localization signal derived from the SV40 virus and in which the C-terminal of the SpCas9 protein was linked to a peptide composed of the 54th to 80th of the Cdtl protein derived from a mouse was prepared. FIG. 8G illustrates the structure of the pX330-pNCmC vector.

### Experiment Example 9

### Study 1 of Subcellular Localization of Cas9 Fusion Protein

HEK293T cells, which are cells derived from human fetal kidney, were used to study subcellular localization of the fusion proteins expressed by the introduction of the expression vectors prepared in Experiment Example 8.

First, the pX330-pFmC vector, the pX330-pCmC vector, the pX330-pMmC vector, the pX330-pNmC vector, the pX330-pNNmC vector, and the pX330-pNCmC vector were introduced to the HEK293T cells, respectively. HEK293T cells with the pX330 vector introduced were also prepared for comparison.

The fusion proteins encoded by these vectors are each linked to a 3×FLAG tag peptide. Thus, each one of the Cas9 proteins can be detected by immunostaining using an anti-FLAG antibody.

Each cell was then formaldehyde-fixed and immunostained with an anti-FLAG antibody. Then, the nuclei were stained with DAPI. Each stained cell was then observed using a fluorescence microscope.

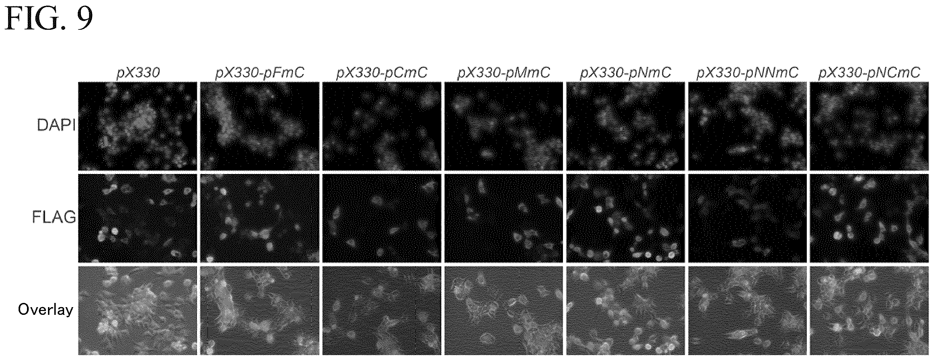

FIG. 9 shows fluorescence micrographs presenting the results of immunostaining. In FIG. 9, "pX330" represents the results of the introduction of the pX330 vector, "pX330-pFmC" represents the results of the introduction of the pX330-pFmC vector, "pX330-pCmC" represents the results of the introduction of the pX330-pCmC vector, "pX330-pMmC" represents the results of the introduction of the pX330-pMmC vector, "pX330-pNmC" represents the results of the introduction of the pX330-pNmC vector, "pX330-pNNmC" represents the results of the introduction of the pX330-pNNmC vector, and "pX330-pNCmC" represents the results of the introduction of the pX330-pNCmC vector. Also, "DAPI" represents the results of staining the nuclei with DAPI, "FLAG" represents the results of detecting each one of the Cas9 proteins by immunostaining using an anti-FLAG antibody, and "Overlay" represents the results of synthesizing the staining results using DAPI and the staining results using the anti-FLAG antibody.

As a result, it was found that the Cas9-pFmC fusion protein and the Cas9-pNmC fusion protein were localized in the nucleus. In contrast, the SpCas9 protein, the Cas9-pCmC fusion protein, the Cas9-pMmC fusion protein, the Cas9-pNNmC fusion protein and the Cas9-pNCmC fusion protein expressed by introducing the pX330 vector did not show nuclear localization.

### Experiment Example 10

### Study 2 of Subcellular Localization of Cas9 Fusion Protein

Soluble nuclear protein fractions and cytoplasmic protein fractions were separately extracted from the HEK293T cells in which each one of the fusion proteins was expressed in Experiment Example 9 using a commercially available kit (Code No. "295-73901", Wako Pure Chemical Industries, Ltd.), and each one of the Cas9 fusion proteins was then detected using Western blotting.

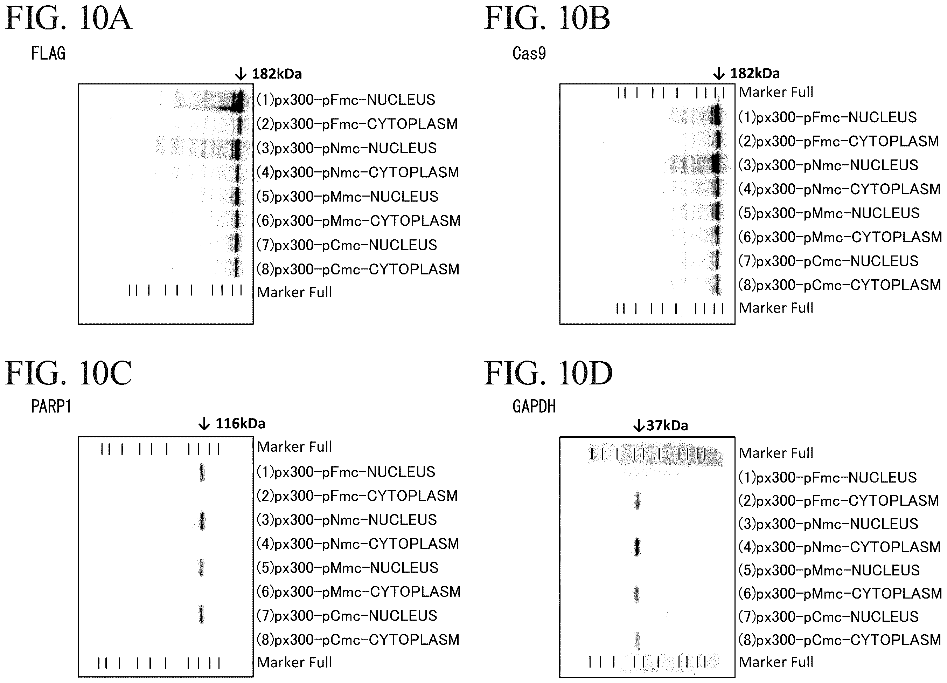

FIG. 10A is a photograph presenting the results of detecting each one of the Cas9 fusion proteins using an anti-FLAG antibody. In addition, FIG. 10B is a photograph showing the results of detecting each one of the Cas9 fusion proteins using an anti-Cas9 antibody. As a result, similar to the result of Experiment Example 9, it was confirmed that more of the Cas9-pFmC fusion protein and the Cas9-pNmC fusion protein were detected in the nucleus rather than the cytoplasm and that the Cas9-pFmC fusion protein and the Cas9-pNmC fusion protein were localized in the nucleus.

FIG. 10C is a photograph presenting the results of detecting the nuclear protein PARP1, as a control, using an anti-PARP1 antibody. FIG. 10D is a photograph showing the results of detecting the cytoplasmic protein GAPDH, as a control, using an anti-GAPDH antibody. As a result, it was confirmed that the protein fractions and cytoplasmic fractions were able to be extracted.

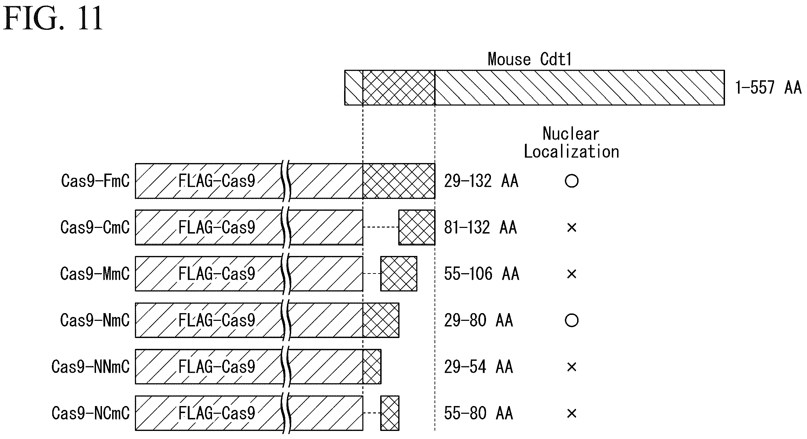

FIG. 11 is a chart summarizing the structure and nuclear localization of each one of the Cas9 fusion proteins based on the results of Experiment Examples 9 and 10. In FIG. 11, "○" indicates high nuclear localization, while "×" indicates that no nuclear localization was observed.

### Experiment Example 11

### Study of Degradability of Cas9 Fusion Protein

HEK293T cells, which are cells derived from human fetal kidney, were used to study degradability of the Cas9-pFmC fusion protein and the Cas9-pNmC fusion protein prepared in Experiment Example 8.

First, the pX330-pFmC vector and the pX330-pNmC vector were introduced into HEK293T cells, respectively. HEK293T cells with the pX330 vector introduced were also prepared for comparison.

Each cell was then formaldehyde-fixed and immunostained with an anti-FLAG antibody. Then, the nuclei were stained with DAPI. Each stained cell was then observed using a fluorescence microscope.

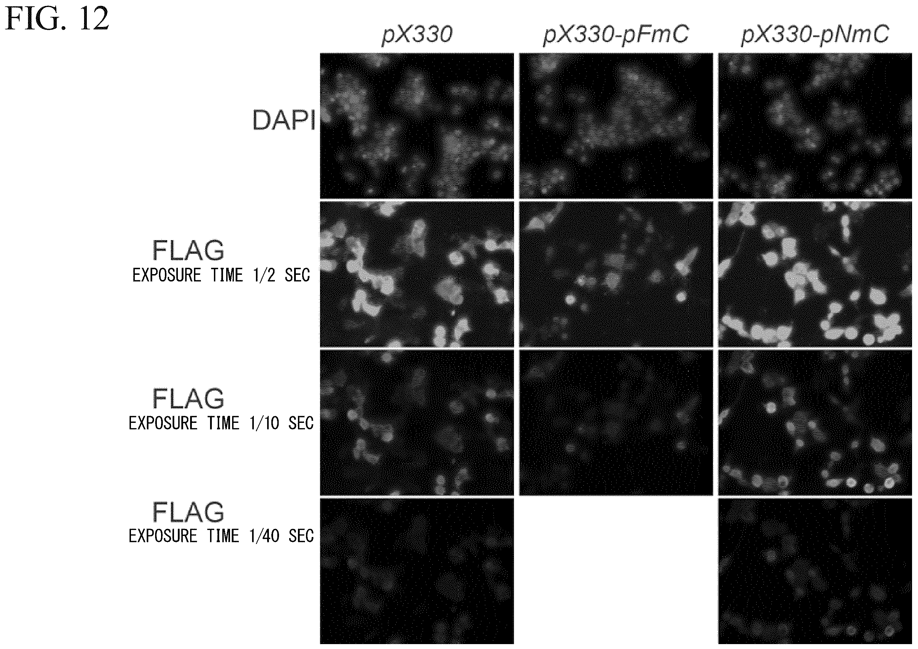

FIG. 12 shows fluorescence micrographs presenting the results of immunostaining. In FIG. 9, "pX330" represents the results of the introduction of the pX330 vector, "pX330-pFmC" represents the results of the introduction of the pX330-pFmC vector, and "pX330-pNmC" represents the results of the introduction of the pX330-pNmC vector. Also, "DAPI" represents the results of staining the nuclei with DAPI, while "FLAG" represents the results of detecting each one of the Cas9 proteins by immunostaining using an anti-FLAG antibody.

As a result, the Cas9-pNmC fusion protein was detected with higher fluorescence intensity of the anti-FLAG antibody than the Cas9-pFmC fusion protein. This result indicates that the Cas9-pNmC fusion protein is less likely to be degraded than the Cas9-pFmC fusion protein.

### Industrial Applicability

According to an embodiment of the present invention, a modified Cas9 that improves KO efficiency and KI efficiency can be provided.

## Claims

1. A fusion protein comprising a Cas9 protein and a modifying peptide that modifies the Cas9 protein, wherein the modifying peptide includes: a peptide consists of the amino acid sequence set forth in SEQ ID NO: 29; or a peptide consists of an amino acid sequence in which one or several amino acids are deleted, substituted, or added to the amino acid sequence set forth in SEQ ID NO: 29 and having activity to localize the Cas9 protein in the nucleus by forming a fusion protein with the Cas9 protein.

2. The fusion protein according to Claim 1, wherein the modifying peptide comprises: a peptide consists of the amino acid sequence set forth in SEQ ID NO: 1; or a peptide consists of an amino acid sequence in which one or several amino acids are deleted, substituted, or added to the amino acid sequence set forth in SEQ ID NO: 1 and having activity to localize the Cas9 protein in the nucleus by forming a fusion protein with the Cas9 protein.

3. The fusion protein according to Claim 1 or 2, wherein the modifying peptide further comprises: a peptide consists of the amino acid sequence set forth in SEQ ID NO: 2; or a peptide consists of an amino acid sequence in which one or several amino acids are deleted, substituted, or added to the amino acid sequence set forth in SEQ ID NO: 2 and having activity to degrade the Cas9 protein in the Gi phase by forming a fusion protein with the Cas9 protein.

4. The fusion protein according to any one of Claims 1 to 3, wherein the modifying peptide is provided at the N-terminal, at the C-terminal, or at a site that is neither the N-terminal nor the C-terminal of the Cas9 protein.

5. A nucleic acid encoding the fusion protein according to any one of Claims 1 to 4.

6. A method for producing a cell in which the genomic DNA are edited, the method comprising contacting the fusion protein according to any one of Claims 1 to 4 with the genomic DNA in a cell.

7. The method according to Claim 6, wherein the cell is a fertilized egg.

8. A method of producing an animal in which the genomic DNA are edited, the method comprising contacting the fusion protein according to any one of Claims 1 to 4 with genomic DNA in a fertilized egg to obtain a fertilized egg in which the genomic DNA are edited, and growing the fertilized egg in which the genomic DNA are edited into an individual to obtain the animal in which the genomic DNA are edited.
